# EUROPEAN PATENT SPECIFICATION

(11) **EP 2 571 849 B1**
(45) Date of publication and mention of the grant of the patent: **29.10.2014**
(21) Application number: 11721956.8
(22) Date of filing: 18.05.2011
(51) Int. Cl.: C07D 207/27, C07D 207/267

(54) **PROCESSES FOR THE PRODUCTIONS OF PYRROLIDONES**
VERFAHREN ZUR HERSTELLUNG VON PYRROLIDONEN
PROCÉDÉS DE FABRICATION DE PYRROLIDONES

(30) Priority: 19.05.2010 US 346135 P
(43) Date of publication of application: 27.03.2013
(73) Proprietor: BioAmber S.A.S., 51110 Bazancourt (FR)
(72) Inventor: FRUCHEY, Olan, S., Hurricane WV 25526 (US); MANZER, Leo, E., Willmington DE 19803 (US); DUNUWILA, Dilum, Princeton NJ 08540 (US); KEEN, Brian, T., Pinch WV 25156 (US); ALBIN, Brooke, A., Charleston WV 25303 (US); CLINTON, Nye, A., Hurricane WV 25526 (US); DOMBEK, Bernard, D., Charleston WV 25314 (US)
(74) Representative: Patentanwälte Ruff, Wilhelm, Beier, Dauster & Partner
(86) International application number: PCT/US2011/036919
(87) International publication number: WO 2011/146561

(56) References cited:
- JP-A- 2005 139 156
- US-A1- 2010 044 626

## Description

### Technical Field

This disclosure relates to processes for producing nitrogen containing compounds such as pyrrolidones from succinic acid (SA) and monoammonium succinate (MAS) produced by fermentation.

### Background

Certain carbonaceous products of sugar fermentation are seen as replacements for petroleum-derived materials for use as feedstocks for the manufacture of carbon-containing chemicals. One such product is MAS.

A material related to MAS, namely SA, can be produced by microorganisms using fermentable carbon sources such as sugars as starting materials. However, most commercially viable, succinate producing microorganisms described in the literature neutralize the fermentation broth to maintain an appropriate pH for maximum growth, conversion and productivity. Typically, the pH of the fermentation broth is maintained at or near a pH of 7 by introduction of ammonium hydroxide into the broth, thereby converting the SA to diammonium succinate (DAS). The DAS must be converted to MAS to derive MAS from the fermentation broth. In a further step, MAS can be converted to SA.

Kushiki (Japanese Published Patent Application, Publication No. 2005-139156) discloses a method of obtaining MAS from an aqueous solution of DAS that could be obtained from a fermentation broth to which an ammonium salt is added as a counter ion. Specifically, MAS is crystallized from an aqueous solution of DAS by adding acetic acid to the solution to adjust the pH of the solution to a value between 4.6 and 6.3, causing impure MAS to crystallize from the solution.

Masuda (Japanese Unexamined Application Publication P2007-254354, Oct. 4, 2007) describes partial deammoniation of dilute aqueous solutions of "ammonium succinate" of the formula H₄NOOCCH₂CH₂COONH₄. From the molecular formula disclosed, it can be seen that "ammonium succinate" is diammonium succinate. Masuda removes water and ammonia by heating solutions of the ammonium succinate to yield a solid SA-based composition containing, in addition to ammonium succinate, at least one of MAS, SA, monoamide succinate, succinimide, succinamide or ester succinate. Thus, it can be inferred that like Kushiki, Masuda discloses a process that results in production of impure MAS. The processes of both Kushiki and Masuda lead to materials that need to be subjected to various purification regimes to produce high purity MAS.

Fischer et al. (US 2010/0044626 A1) discloses a process for the preparation of pyrrolidinones comprising the steps of providing a diammonium succinate-containing fermentation broth, distilling the broth, distilling under conditions such that succinimide is formed, and hydrogenating the succinimide into a pyrrolidinone.

Bio-derived SA compounds such as those derived from MAS and/or DAS are platform molecules for synthesis of a number of commercially important chemicals and polymers. Therefore, it is highly desirable to provide a purification technology that offers flexibility to integrate clear, commercially viable paths to derivatives such as pyrrolidones. Pyrrolidones have a wide variety of important industrial applications, Thus, in response to the lack of economically and technically viable process solutions for converting fermentation-derived SA compounds to pyrrolidones, it could be helpful to provide methods for providing a cost effective SA compound stream of sufficient purity for direct production that may be used for the production of such pyrrolidones.

### Summary

We provide a process for making nitrogen containing compounds, including (a) providing a clarified DAS-containing fermentation broth; (b) distilling the broth to form an overhead that comprises water and ammonia, and a liquid bottoms that comprises MAS, at least some DAS, and at least about 20 wt% water; (c) cooling and/or evaporating the bottoms, and optionally adding an antisolvent to the bottoms, to attain a temperature and composition sufficient to cause the bottoms to separate into a DAS-containing liquid portion and a MAS-containing solid portion that is substantially free of DAS; (d) separating at least part of the solid portion from the liquid portion; and (e) (1) contacting at least a part of the solid portion with hydrogen and, optionally an ammonia source, in the presence of a hydrogenation catalyst at a temperature of about 150°C to about 400°C and a pressure of about 0.68 to about 27.6 MPa to produce the compound of Formula I; or (2) contacting at least a part of the solid portion with hydrogen and either an alkylamine of the formula R-NH₂ or an alcohol of the formula R-OH, wherein R is a linear or branched C₁ to C₂₀ alkyl group or a C₅ to C₂₀ substituted or unsubstituted cycloalkyl group or an aromatic group C₆ or larger, and, optionally an ammonia source, in the presence of a hydrogenation catalyst at a temperature of about 150°C to about 400°C and a pressure of about 0.68 to about 27.6 MPa to produce the compound of Formula II; or (3) contacting at least a part of the solid portion with hydrogen and NH₂CH₂CH₂OH or ethylene glycol and hydrogen and, optionally an ammonia source, in the presence of a hydrogenation catalyst at a temperature of about 150°C to about 400°C and a pressure of about 0.68 to about 27.6 MPa to produce the compound of Formula III; and (f) recovering the compounds of Formula I, Formula II or Formula III

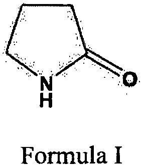

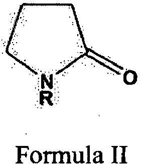

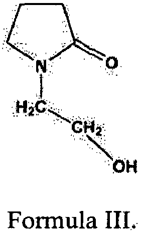

We also provide a process for making nitrogen containing compounds, including (a) providing a clarified DAS-containing fermentation broth; (b) distilling the broth to form a first overhead that includes water and ammonia, and a first liquid bottoms that includes MAS, at least some DAS, and at least about 20 wt% water; (c) cooling and/or evaporating the bottoms, and optionally adding an antisolvent to the bottoms, to attain a temperature and composition sufficient to cause the bottoms to separate into a DAS-containing liquid portion and a MAS-containing solid portion that is substantially free of DAS; (d) separating the solid portion from the liquid portion; (e) recovering the solid portion; (f) dissolving the solid portion in water to produce an aqueous MAS solution; (g) distilling the aqueous MAS solution at a temperature and pressure sufficient to form a second overhead that includes water and ammonia, and a second bottoms that includes a major portion of SA, a minor portion of MAS, and water; (h) cooling and/or evaporating the second bottoms to cause the second bottoms to separate into a second liquid portion in contact with a second solid portion that preferably consists essentially of SA and is substantially free of MAS; (i) separating at least part of the second solid portion from the second liquid portion; and (j) (1) contacting at least a part of the solid portion with hydrogen and, optionally an ammonia source, in the presence of a hydrogenation catalyst at a temperature of about 150°C to about 400°C and a pressure of about 0.68 to about 27.6 MPa to produce the compound of Formula I; or (2) contacting at least a part of the solid portion with hydrogen and either an alkylamine of the formula R-NH₂ or an alcohol of the formula R-OH, wherein R is a linear or branched C₁ to C₂₀ alkyl group or a C₅ to C₂₀ substituted or unsubstituted cycloalkyl group or an aromatic group C₆ or larger, and, optionally an ammonia source, in the presence of a hydrogenation catalyst at a temperature of about 150°C to about 400°C and a pressure of about 0.68 to about 27.6 MPa to produce the compound of Formula II; or (3) contacting at least a part of the solid portion with hydrogen and NH₂CH₂CH₂OH or ethylene glycol and hydrogen and, optionally an ammonia source, in the presence of a hydrogenation catalyst at a temperature of about 150°C to about 400°C and a pressure of about 0.68 to about 27.6 MPa to produce the compound of Formula III; and (k) recovering the compounds of Formula I, Formula II or Formula III.

We further provide a process for making nitrogen containing compounds, including (a) providing a clarified MAS-containing fermentation broth; (b) optionally, adding MAS, DAS, SA, NH₃, and/or NH₄⁺ to the broth to preferably maintain the pH of the broth below 6; (c) distilling the broth to form an overhead that includes water and optionally ammonia, and a liquid bottoms that includes MAS, at least some DAS, and at least about 20 wt% water; (d) cooling and/or evaporating the bottoms, and optionally adding an antisolvent to the bottoms, to attain a temperature and composition sufficient to cause the bottoms to separate into a DAS-containing liquid portion and a MAS-containing solid portion that is substantially free of DAS; (e) separating at least part of the solid portion from the liquid portion; and (f) (1) contacting at least a part of the solid portion with hydrogen and, optionally an ammonia source, in the presence of a hydrogenation catalyst at a temperature of about 150°C to about 400°C and a pressure of about 0.68 to about 27.6 MPa to produce the compound of Formula I; or (2) contacting at least a part of the solid portion with hydrogen and either an alkylamine of the formula R-NH₂ or an alcohol of the formula R-OH, wherein R is a linear or branched C₁ to C₂₀ alkyl group or a C₅ to C₂₀ substituted or unsubstituted cycloalkyl group or an aromatic group C₆ or larger, and, optionally an ammonia source, in the presence of a hydrogenation catalyst at a temperature of about 150°C to about 400°C and a pressure of about 0.68 to about 27.6 MPa to produce the compound of Formula II; or (3) contacting at least a part of the solid portion with hydrogen and NH₂CH₂CH₂OH or ethylene glycol and hydrogen and, optionally an ammonia source, in the presence of a hydrogenation catalyst at a temperature of about 150°C to about 400°C and a pressure of about 0.68 to about 27.6 MPa to produce the compound of Formula III; and (g) recovering the compounds of Formula I, Formula II or Formula III.

We further yet provide a process for making nitrogen containing compounds, including (a) providing a clarified MAS-containing fermentation broth; (b) optionally, adding MAS, DAS, SA, NH₃, and/or NH₄⁺ to the broth to preferably maintain the pH of the broth below 6; (c) distilling the broth to form an overhead that includes water and optionally ammonia, and a liquid bottoms that includes MAS, at least some DAS, and at least about 20 wt% water; (d) cooling and/or evaporating the bottoms, and optionally adding an antisolvent to the bottoms, to attain a temperature and composition sufficient to cause the bottoms to separate into a DAS-containing liquid portion and a MAS-containing solid portion that is substantially free of DAS; (e) separating the solid portion from the liquid portion; and (f) recovering the solid portion; (g) dissolving the solid portion in water to produce an aqueous MAS solution; (h) distilling the aqueous MAS solution at a temperature and pressure sufficient to form a second overhead that includes water and ammonia, and a second bottoms that includes a major portion of SA, a minor portion of MAS, and water; (i) cooling and/or evaporating the second bottoms to cause the second bottoms to separate into a second liquid portion in contact with a second solid portion that preferably consists essentially of SA and is substantially free of MAS; (j) separating at least part of the second solid portion from the second liquid portion; and (k) (1) contacting at least a part of the solid portion with hydrogen and, optionally an ammonia source, in the presence of a hydrogenation catalyst at a temperature of about 150°C to about 400°C and a pressure of about 0.68 to about 27.6 MPa to produce the compound of Formula I; or (2) contacting at least a part of the solid portion with hydrogen and either an alkylamine of the formula R-NH₂ or an alcohol of the formula R-OH, wherein R is a linear or branched C₁ to C₂₀ alkyl group or a C₅ to C₂₀ substituted or unsubstituted cycloalkyl group or an aromatic group C₆ or larger, and, optionally an ammonia source, in the presence of a hydrogenation catalyst at a temperature of about 150°C to about 400°C and a pressure of about 0.68 to about 27.6 MPa to produce the compound of Formula II; or (3) contacting at least a part of the solid portion with hydrogen and NH₂CH₂CH₂OH or ethylene glycol and hydrogen and, optionally an ammonia source, in the presence of a hydrogenation catalyst at a temperature of about 150°C to about 400°C and a pressure of about 0.68 to about 27.6 MPa to produce the compound of Formula III; and (1) recovering the compounds of Formula I, Formula II or Formula III.

We still further provide a process which additionally includes contacting the compound of Formula I with acetylene in the presence of a basic catalyst at a temperature of about 80°C to about 250°C and a pressure of about 0.5 to about 25 MPa to produce the compound of Formula IV

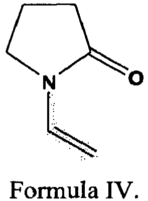

We yet further provide a process which additionally includes dehydrating the compound of Formula III at a temperature of about 100°C to about 500°C and a pressure of about 0.068 to about 1.37 MPa to produce the compound of Formula IV

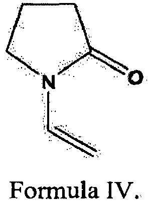

### Brief Description of the Drawings

Fig. 1 schematically illustrates a fully integrated process for production of fermentation-derived MAS and SA and their further conversion to pyrrolidones and depicts two-stage deammoniation of DAS with a MAS crystallization step between the two stages.
Fig. 2 schematically illustrates examples of selected pyrrolidones produced from MAS.
Fig. 3 schematically illustrates examples of selected pyrrolidones produced from SA.
Fig. 4 is a graph showing the solubility of MAS as a function of temperature in both water and a 30% aqueous DAS solution.

### Detailed Description

It will be appreciated that at least a portion of the following description is intended to refer to representative examples of processes selected for illustration in the drawings and is not intended to define or limit the disclosure, other than in the appended claims.

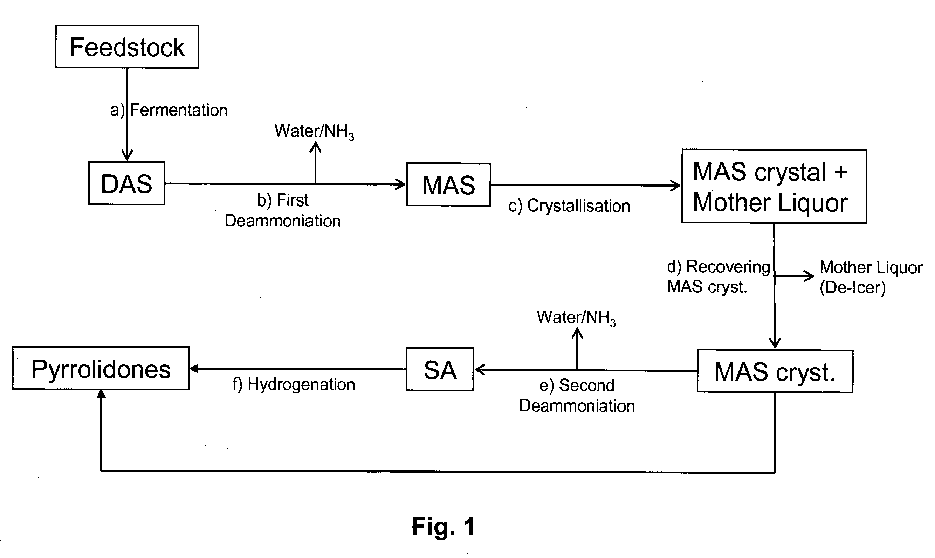

Our processes may be appreciated by reference to Fig. 1, which shows in flow diagram form one representative example of our methods.

A growth vessel, typically an in-place steam sterilizable fermentor, may be used to grow a microbial culture (not shown) that is subsequently utilized for the production of the DAS, MAS, and/or SA -containing fermentation broth. Such growth vessels are known in the art and are not further discussed.

The microbial culture may comprise microorganisms capable of producing SAs from fermentable carbon sources such as carbohydrate sugars. Representative examples of microorganisms include , *Escherichia coli (E. coli), Aspergillus niger, Corynebacterium glutamicum* (also called *Brevibacterium flavum), Enterococcus faecalis, Veillonella parvula, Actinobacillus succinogenes, Mannheimia succiniciproducens, Anaerobiospirillum succiniciproducens, Paecilomyces Varioti, Saccharomyces cerevisiae, Bacteroides fragilis, Bacteroides ruminicola, Bacteroides amylophilus, Alcaligenes eutrophus, Brevibacterium ammoniagenes, Brevibacterium lactofermentum, Candida brumptii, Candida catenulate, Candida mycoderma, Candida zeylanoides, Candida paludigena, Candida sonorensis, Candida utilis, Candida zeylanoides, Debaryomyces hansenii, Fusarium oxysporum, Humicola lanuginosa, Kloeckera apiculata, Kluyveromyces lactis, Kluyveromyces wickerhamii, Penicillium simplicissimum, Pichia anomala, Pichia besseyi, Pichia media, Pichia guilliermondii, Pichia inositovora, Pichia stipidis, Saccharomyces bayanus, Schizosaccharomyces pombe, Torulopsis candida, Yarrowia lipolytica,* mixtures thereof and the like.

A preferred microorganism is an *E. coli* strain deposited at the ATCC under accession number PTA-5132. More preferred is this strain with its three antibiotic resistance genes *(cat, amphl, tetA)* removed. Removal of the antibiotic resistance genes *cat* (coding for the resistance to chloramphenicol), and *amphl* (coding for the resistance to kanamycin) can be performed by the so-called "Lambda-red (λ-red)" procedure as described in Datsenko KA and Wanner BL., Proc. Natl. Acad. Sci. USA 2000 Jun 6; 97(12) 6640-5. The tetracycline resistant gene *tetA* can be removed using the procedure originally described by Bochner et al., J Bacteriol. 1980 August; 143(2): 926-933, the subject matter of which is incorporated herein by reference. Glucose is a preferred fermentable carbon source for this microorganism.

A fermentable carbon source (e.g., carbohydrates and sugars), optionally a source of nitrogen and complex nutrients (e.g., corn steep liquor), additional media components such as vitamins, salts and other materials that can improve cellular growth and/or product formation, and water may be fed to the growth vessel for growth and sustenance of the microbial culture. Typically, the microbial culture is grown under aerobic conditions provided by sparging an oxygen-rich gas (e.g., air or the like). Typically, an acid (e.g., sulphuric acid or the like) and ammonium hydroxide are provided for pH control during the growth of the microbial culture.

In one example (not shown), the aerobic conditions in the growth vessel (provided by sparging an oxygen-rich gas) are switched to anaerobic conditions by changing the oxygen-rich gas to an oxygen-deficient gas (e.g., CO₂ or the like). The anaerobic environment triggers bioconversion of the fermentable carbon source to SA *in situ* in the growth vessel. Ammonium hydroxide is provided for pH control during bioconversion of the fermentable carbon source to SA. The SA that is produced is at least partially neutralized to DAS due to the presence of the ammonium hydroxide, leading to the production of a broth comprising DAS. The CO₂ provides an additional source of carbon for the production of SA.

In another example, the contents of the growth vessel may be transferred via a stream to a separate bioconversion vessel for bioconversion of a carbohydrate source to SA. An oxygen-deficient gas (e.g., CO₂ or the like) is sparged in the bioconversion vessel to provide anaerobic conditions that trigger production of SA. Ammonium hydroxide is provided for pH control during bioconversion of the carbohydrate source to SA. Due to the presence of the ammonium hydroxide, the SA produced is at least partially neutralized to DAS, leading to production of a broth that comprises DAS. The CO₂ provides an additional source of carbon for production of SA.

In another example, the bioconversion may be conducted at relatively low pH (e.g., 3 to 6). A base (ammonium hydroxide or ammonia) may be provided for pH control during bioconversion of the carbohydrate source to SA. Depending of the desired pH, due to the presence or lack of the ammonium hydroxide, either SA is produced or the SA produced is at least partially neutralized to MAS, DAS, or a mixture comprising SA, MAS and/or DAS. Thus, the SA produced during bioconversion can be subsequently neutralized, optionally in an additional step, by providing either ammonia or ammonium hydroxide leading to a broth comprising DAS. As a consequence, a "DAS-containing fermentation broth" generally means that the fermentation broth comprises DAS and possibly any number of other components such as MAS and/or SA, whether added and/or produced by bioconversion or otherwise. Similarly, a "MAS-containing fermentation broth" generally means that the fermentation broth comprises MAS and possibly any number of other components such as DAS and/or SA, whether added and/or produced by bioconversion or otherwise.

The broth resulting from the bioconversion of the fermentable carbon source (in either the growth vessel or the bioconversion vessel, depending on where the bioconversion takes place), typically contains insoluble solids such as cellular biomass and other suspended material, which are transferred via a stream to a clarification apparatus before distillation. Removal of insoluble solids clarifies the broth. This reduces or prevents fouling of subsequent distillation equipment. The insoluble solids can be removed by any one of several solid-liquid separation techniques, alone or in combination, including but not limited to, centrifugation and filtration (including, but not limited to ultra-filtration, micro-filtration or depth filtration). The choice of filtration can be made using techniques known in the art. Soluble inorganic compounds can be removed by any number of known methods such as, but not limited to, ion-exchange, physical adsorption and the like.

An example of centrifugation is a continuous disc stack centrifuge. It may be useful to add a polishing filtration step following centrifugation such as dead-end or crossflow filtration that may include the use of a filter aide such as diatomaceous earth or the like, or more preferably ultra-filtration or micro-filtration. The ultra-filtration or micro-filtration membrane can be ceramic or polymeric, for example. One example of a polymeric membrane is SelRO MPS-U20P (pH stable ultra-filtration membrane) manufactured by Koch Membrane Systems (850 Main Street, Wilmington, MA, USA). This is a commercially available polyethersulfone membrane with a 25,000 Dalton molecular weight cut-off which typically operates at pressures of 0.35 to 1.38 MPa (maximum pressure of 1.55 MPa) and at temperatures up to 50°C. Alternatively, a filtration step may be employed, such as ultra-filtration or micro-filtration alone.

The resulting clarified DAS-containing broth or MAS-containing broth, substantially free of the microbial culture and other solids, is transferred via a stream to a distillation apparatus.

The clarified distillation broth should contain DAS and/or MAS in an amount that is at least a majority of, preferably at least about 70 wt%, more preferably 80 wt% and most preferably at least about 90 wt% of all the diammonium dicarboxylate salts in the broth. The concentration of DAS and/or MAS as a weight percent (wt%) of the total dicarboxylic acid salts in the fermentation broth can be easily determined by high pressure liquid chromatography (HPLC) or other known means.

Water and ammonia are removed from the distillation apparatus as an overhead, and at least a portion is optionally recycled via a stream to the bioconversion vessel (or the growth vessel operated in the anaerobic mode). Distillation temperature and pressure are not critical as long as the distillation is carried out in a way that ensures that the distillation overhead contains water and ammonia, and the distillation bottoms comprises at least some DAS and at least about 20 wt% water. A more preferred amount of water is at least about 30 wt% and an even more preferred amount is at least about 40 wt%. The rate of ammonia removal from the distillation step increases with increasing temperature and also can be increased by injecting steam (not shown) during distillation. The rate of ammonia removal during distillation may also be increased by conducting distillation under a vacuum or by sparging the distillation apparatus with a non-reactive gas such as air, nitrogen or the like.

Removal of water during the distillation step can be enhanced by the use of an organic azeotroping agent such as toluene, xylene, cyclohexane, methyl cyclohexane, methyl isobutyl ketone, heptane or the like, provided that the bottoms contains at least about 20 wt% water. If the distillation is carried out in the presence of an organic agent capable of forming an azeotrope consisting of the water and the agent, distillation produces a biphasic bottoms that comprises an aqueous phase and an organic phase, in which case the aqueous phase can be separated from the organic phase, and the aqueous phase used as the distillation bottoms. Byproducts such as succinamic acid, succinamide and succinimide are substantially avoided provided the water level in the bottoms is maintained at a level of at least about 30 wt%.

A preferred temperature for the distillation step is in the range of about 50°C to about 300°C, depending on the pressure. A more preferred temperature range is about 90°C to about 150°C, depending on the pressure. A distillation temperature of about 110°C to about 140°C is preferred. "Distillation temperature" refers to the temperature of the bottoms (for batch distillations this may be the temperature at the time when the last desired amount of overhead is taken).

Adding a water miscible organic solvent or an ammonia separating solvent facilitates deammoniation over a variety of distillation temperatures and pressures as discussed above. Such solvents include aprotic, bipolar, oxygen-containing solvents that may be able to form passive hydrogen bonds. Examples include, but are not limited to, diglyme, triglyme, tetraglyme, propylene glycol, sulfoxides such as dimethylsulfoxide (DMSO), lactones such as gamma-butyrolactonce (GBL), amides such as dimethylformamide (DMF) and dimethylacetamide, sulfones such as dimethylsulfone, sulfolane, polyethyleneglycol (PEG), butoxytriglycol, N-methylpyrolidone (NMP), ethers such as dioxane, methyl ethyl ketone (MEK) and the like. Such solvents aid in the removal of ammonia from the DAS or MAS in the clarified broth. Regardless of the distillation technique, it is important that the distillation be carried out in a way that ensures that at least some DAS and at least about 20 wt% water remain in the bottoms and even more advantageously at least about 30 wt%.

The distillation can be performed at atmospheric, sub-atmospheric or superatmospheric pressures. The distillation can be a one-stage flash, a multistage distillation (i.e., a multistage column distillation) or the like. The one-stage flash can be conducted in any type of flasher (e.g., a wiped film evaporator, thin film evaporator, thermosiphon flasher, forced circulation flasher and the like). The multistages of the distillation column can be achieved by using trays, packing or the like. The packing can be random packing (e.g., Raschig rings, Pall rings, Berl saddles and the like) or structured packing (e.g., Koch-Sulzer packing, Intalox packing, Mellapak and the like). The trays can be of any design (e.g., sieve trays, valve trays, bubble-cap trays and the like). The distillation can be performed with any number of theoretical stages.

If the distillation apparatus is a column, the configuration is not particularly critical, and the column can be designed using well known criteria. The column can be operated in either stripping mode, rectifying mode or fractionation mode. Distillation can be conducted in either batch or continuous mode. In the continuous mode, the broth is fed continuously into the distillation apparatus, and the overhead and bottoms are continuously removed from the apparatus as they are formed. The distillate from distillation is an ammonia/water solution, and the distillation bottoms is a liquid, aqueous solution of MAS and DAS, which may also contain other fermentation by-product salts (i.e., ammonium acetate, ammonium formate, ammonium lactate and the like) and color bodies.

The distillation bottoms can be transferred via a stream to a cooling apparatus and cooled by conventional techniques. Cooling technique is not critical. A heat exchanger (with heat recovery) can be used. A flash vaporization cooler can be used to cool the bottoms to about 15°C. Cooling to <15°C typically employs a refrigerated coolant such as, for example, glycol solution or, less preferably, brine. A concentration step can be included prior to cooling to help increase product yield. Further, both concentration and cooling can be combined using methods known such as vacuum evaporation and heat removal using integrated cooling jackets and/or external heat exchangers.

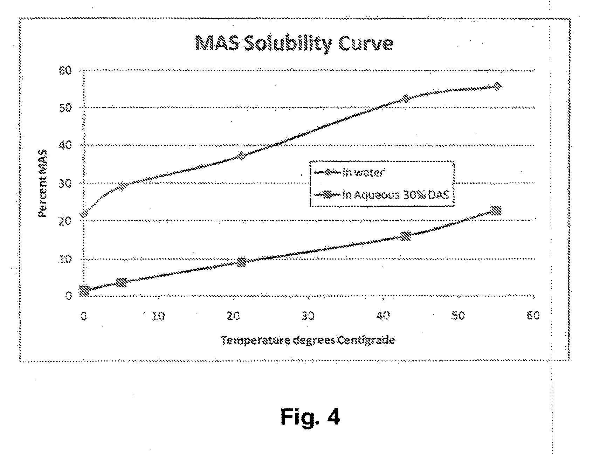

We found that the presence of some DAS in the liquid bottoms facilitates cooling-induced separation of the bottoms into a liquid portion in contact with a solid portion that at least "consists essentially" of MAS (meaning that the solid portion is at least substantially pure crystalline MAS) by reducing the solubility of MAS in the liquid, aqueous, DAS-containing bottoms. Fig. 4 illustrates the reduced solubility of MAS in an aqueous 30 wt% DAS solution at various temperatures ranging from 0°C to 60°C. The upper curve shows that even at 0°C MAS remains significantly soluble in water (i.e., about 20 wt% in aqueous solution). The lower curve shows that at 0°C MAS is essentially insoluble in a 30 wt% aqueous DAS solution. We discovered, therefore, that MAS can be more completely crystallized out of an aqueous solution if some DAS is also present in that solution. A preferred concentration of DAS in such a solution is in the ppm to about 3 wt% range. This allows crystallization of MAS (i.e., formation of the solid portion of the distillation bottoms) at temperatures higher than those that would be required in the absence of DAS.

When about 50% of the ammonia is removed from DAS contained in an aqueous medium the succinate species establish an equilibrium molar distribution that is about 0.1:0.8:0.1 in DAS:MAS:SA within a pH range of 4.8 to 5.4, depending on the operating temperature and pressure. When this composition is concentrated and cooled, MAS exceeds its solubility limit in water and crystallizes. When MAS undergoes a phase change to the solid phase, the liquid phase equilibrium resets thereby producing more MAS (DAS donates an ammonium ion to SA). This causes more MAS to crystallize from solution and continues until appreciable quantities of SA are exhausted and the pH tends to rise. As the pH rises, the liquid phase distribution favors DAS. However, since DAS is highly soluble in water, MAS continues to crystallize as its solubility is lower than DAS. In effect, the liquid phase equilibrium and the liquid-solid equilibria of succinate species act as a "pump" for MAS crystallization, thereby enabling MAS crystallization in high yield.

In addition to cooling, evaporation, or evaporative cooling described above, crystallization of MAS can be enabled and/or facilitated by addition of an antisolvent. In this context, antisolvents may be solvents typically miscible with water, but cause crystallization of a water soluble salt such as MAS due to lower solubility of the salt in the solvent. Solvents with an antisolvent effect on MAS can be alcohols such as ethanol and propanol, ketones such as methyl ethyl ketone, ethers such as tetrahydrofuran and the like. The use of antisolvents is known and can be used in combination with cooling and evaporation or separately.

The distillation bottoms, after cooling in the cooling unit, is fed via a stream to a separator for separation of the solid portion from the liquid portion. Separation can be accomplished via pressure filtration (e.g., using Nutsche or Rosenmond type pressure filters), centrifugation and the like. The resulting solid product can be recovered as a product and dried, if desired, by standard methods.

After separation, it may be desirable to treat the solid portion to ensure that no liquid portion remains on the surface(s) of the solid portion. One way to minimize the amount of liquid portion that remains on the surface of the solid portion is to wash the separated solid portion with water and dry the resulting washed solid portion. A convenient way to wash the solid portion is to use a so-called "basket centrifuge." Suitable basket centrifuges are available from The Western States Machine Company (Hamilton, OH, USA).

The liquid portion of the distillation bottoms (i.e., the mother liquor) may contain remaining dissolved MAS, any unconverted DAS, any fermentation byproducts such as ammonium acetate, lactate, or formate, and other minor impurities. This liquid portion can be fed via a stream to a downstream apparatus. In one instance, the downstream apparatus may be a means for making a de-icer by treating in the mixture with an appropriate amount of potassium hydroxide, for example, to convert the ammonium salts to potassium salts. Ammonia generated in this reaction can be recovered for reuse in the bioconversion vessel (or the growth vessel operating in the anaerobic mode). The resulting mixture of potassium salts is valuable as a de-icer and anti-icer.

The mother liquor from the solids separation step, can be recycled (or partially recycled) to the distillation apparatus via a stream to further enhance recovery of MAS, as well as further convert DAS to MAS.

The solid portion of the cooling-induced crystallization is substantially pure MAS and is, therefore, useful for the known utilities of MAS.

HPLC can be used to detect the presence of nitrogen-containing impurities such as succinamide and succinimide. The purity of MAS can be determined by elemental carbon and nitrogen analysis. An ammonia electrode can be used to determine a crude approximation of MAS purity.

Depending on the circumstances and various operating inputs, there are instances when the fermentation broth may be a clarified MAS-containing fermentation broth or a clarified SA-containing fermentation broth. In those circumstances, it can be advantageous to add MAS, DAS, SA, ammonia and/or ammonium hydroxide to those fermentation broths to facilitate the production of substantially pure MAS. For example, the operating pH of the fermentation broth may be oriented such that the broth is a MAS-containing broth or a SA-containing broth. MAS, DAS, SA, ammonia and/or ammonium hydroxide may be optionally added to those broths to facilitate production of the above-mentioned substantially pure MAS. For example, the operating pH of the fermentation broth may be oriented such that the broth is a MAS-containing broth or a SA-containing broth. MAS, DAS, SA, ammonia, and/or ammonium hydroxide may be optionally added to those broths to attain a broth pH preferably below 6 to facilitate production of the above-mentioned substantially pure MAS. Also, it is possible that MAS, DAS and/or SA from other sources may be added as desired. In one particular form, it is especially advantageous to recycle MAS, DAS and water from the liquid bottoms resulting from the distillation step and/or the liquid portion from the separator into the fermentation broth. In referring to the MAS-containing broth, such broth generally means that the fermentation broth comprises MAS and possibly any number of other components such as DAS and/or SA, whether added and/or produced by bioconversion or otherwise.

The solid portion can be converted into SA by removing ammonia. This can be carried out as follows. The solid portion (consisting essentially of MAS) obtained from any of the above-described conversion processes can be dissolved in water to produce an aqueous MAS solution. This solution can then be distilled at a temperature and pressure sufficient to form an overhead that comprises water and ammonia, and a bottoms that comprises a major portion of SA, a minor portion of MAS and water. The bottoms can be cooled to cause it to separate into a liquid portion in contact with a solid portion that consists essentially of SA and is substantially free of MAS. The solid portion can be separated from the second liquid portion and recovered as substantially pure SA as determined by HPLC.

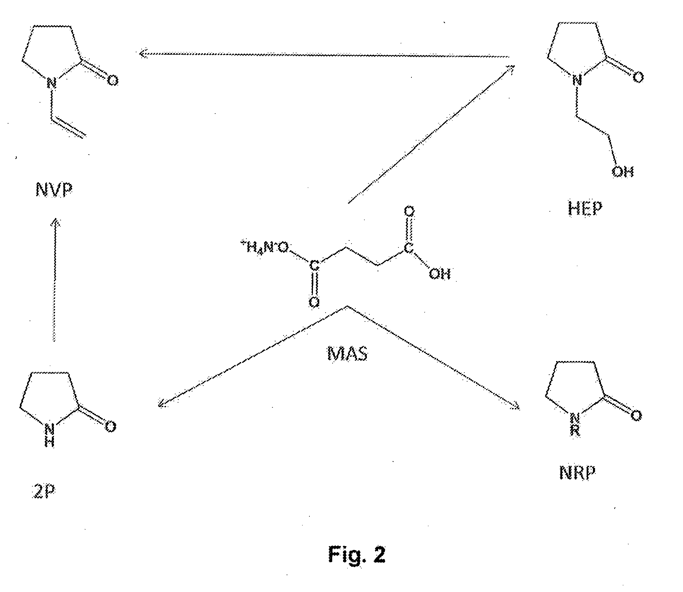

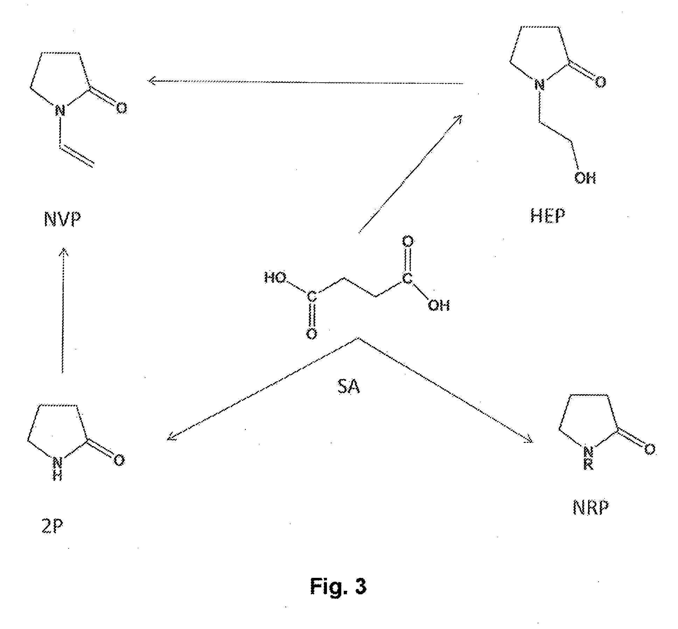

Streams comprising MAS as presented in Fig. 2 and streams comprising SA as presented in Fig. 3 may be contacted with various reactants and a catalyst at selected temperatures and pressures to produce compounds comprising pyrrolidones.

A principal component of the catalyst useful for hydrogenation of SA and MAS may be selected from one or more metals selected from the group consisting of palladium, ruthenium, rhenium, rhodium, iridium, platinum, nickel, cobalt, copper, iron and compounds thereof.

The SA and MAS may be dissolved in water to form an aqueous solution of SA and MAS which can be used for downstream reactions. It is possible to convert such aqueous solutions of SA and MAS to DAS by addition of an ammonia source (e.g., NH₃ or NH₄OH).

Streams comprising traditional source MAS can be converted to 2-pyrrolidone (2P) or N-alkyl-pyrrolidones (NRP) by the reaction of an alkyl-amine, an alcohol or ammonia with hydrogen in the presence of a catalyst as shown in Figs. 2 and 3. In NRP, R typically is a linear or branched C₁-C₂₀ alkyl group or a C₅-C₂₀ substituted or unsubstituted cycloalkyl group or an aromatic group of C₆ or more. Solutions comprising aqueous MAS and methanol can be hydrogenated over Rh/C catalysts to N-methyl-pyrrolidone (NMP) as disclosed in US 6,670,483. For example, US '483 hydrogenates a mixture comprising MAS and methanol with a Rh/C catalyst at 13.2 MPa H2 pressure and a temperature of 265°C. The conversion of MAS was 89.6% converted. The yield of 2P and NMP was 70.9%. This process may be applied to our bio-derived MAS and SA. The use of hydroxy ethanolamine can result in N-2-hydroxyethyl-pyrrolidone (HEP) as shown in Figs. 2 and 3. Use of ammonia in the absence of an alkanol can result in 2P as shown in Figs. 2 and 3.

2P reacts with acetylene to yield N-vinyl pyrrolidone (NVP) as disclosed in Example 1 of US 5,665,889 wherein 2P, KOH and hydroxyl end capped polyether (PTMEG) as a co-catalyst were reacted for 1 hour at 110°C to 115°C under a nitrogen atmosphere to form a potassium salt. A mixture of nitrogen and acetylene was then slowly added to the reaction flash to give NVP 90% yield.

NVP may also be prepared by the catalytic dehydration of HEP. Use of alkali metal catalysts in the gas phase to efficiently carry out the dehydration reaction of HEP is disclosed in US 6,489,515. A solid oxide catalyst contained an alkali metal element to allow reaction to progress by inhibiting decomposition of the raw material and the objective product the catalyst.

US 6,906,200 discloses formation of NVP produced by dehydration of N-hydroxyethyl pyrrolidone in the presence of an amorphous mixed oxide catalyst and obtained 97.8% conversion of HEP to NVP at 348°C in 82% yield using an amorphous Ca/Zn oxide catalyst.

Thus, it is now possible to produce pyrrolidones such as 2P, NRP, HEP and the like by contacting MAS with water and hydrogen in the presence of a hydrogenation catalyst and, optionally, an ammonia source (e.g., NH₃ or NH₄OH) at a temperature of about 150°C to about 400°C and a presence of about 0.68 to about 27.6 MPa. It is also possible to produce pyrrolidones such as 2P, NRP, HEP and the like by contacting SA with an ammonia source and hydrogen in the presence of a hydrogenation catalyst and, optionally, an ammonia source (e.g., NH₃ or NH₄OH) at a temperature of about 150°C to about 400°C and a presence of about 0.68 to about 27.6 MPa.

Hydrogenation catalysts for the conversion of MAS and SA to pyrrolidones may be promoted to augment the activity or selectivity of the catalyst. The promoter may be incorporated into the catalyst during any step in the chemical processing of the catalyst constituent. The chemical promoter generally enhances the physical or chemical function of the catalyst agent, but can also be added to retard undesirable side reactions. Suitable promoters include metals selected from tin, zinc, copper, rhenium, gold, silver, and combinations thereof. Other promoters that can be used are elements selected from Group I and Group II of the Periodic Table.

The catalyst may be supported or unsupported. A supported catalyst is one in which the active catalyst agent is deposited on a support material by a number of methods such as spraying, soaking or physical mixing, followed by drying, calcination and, if necessary, activation through methods such as reduction or oxidation. Materials frequently used as a support are porous solids with high total surface areas (external and internal) which can provide high concentrations of active sites per unit weight of catalyst. The catalyst support may enhance the function of the catalyst agent. A supported metal catalyst is a supported catalyst in which the catalyst agent is a metal.

A catalyst that is not supported on a catalyst support material is an unsupported catalyst. An unsupported catalyst may be platinum black or a Raney@ (W.R. Grace & Co., Columbia, MD) catalyst, for example. Raney® catalysts have a high surface area due to selectively leaching an alloy containing the active metal(s) and a leachable metal (usually aluminum). Raney® catalysts have high activity due to the higher specific area and allow the use of lower temperatures in hydrogenation reactions. The active metals of Raney® catalysts include nickel, copper, cobalt, iron, rhodium, ruthenium, rhenium, osmium, iridium, platinum, palladium, compounds thereof and combinations thereof.

Promoter metals may also be added to the base Raney® metals to affect selectivity and/or activity of the Raney® catalyst. Promoter metals for Raney® catalysts may be selected from transition metals from Groups IIIA through VIIIA, IB and IIB of the Periodic Table of the Elements. Examples of promoter metals include chromium, molybdenum, platinum, rhodium, ruthenium, osmium, and palladium, typically at about 2% by weight of the total metal.

The catalyst support can be any solid, inert substance including, but not limited to, oxides such as silica, alumina and titania; barium sulfate; calcium carbonate; and carbons. The catalyst support can be in the form of powder, granules, pellets or the like.

A preferred support material may be selected from the group consisting of carbon, alumina, silica, silica-alumina, silica-titania, titania, titania-alumina, barium sulfate, calcium carbonate, strontium carbonate, compounds thereof and combinations thereof. Supported metal catalysts can also have supporting materials made from one or more compounds. More preferred supports are carbon, titania and alumina. Further preferred supports are carbons with a surface area greater than about 100 m²/g. A further preferred support is carbon with a surface area greater than about 200 m²/g. Preferably, the carbon has an ash content that is less than about 5% by weight of the catalyst support. The ash content is the inorganic residue (expressed as a percentage of the original weight of the carbon) which remains after incineration of the carbon.

A preferred content of the metal catalyst in the supported catalyst may be from about 0.1% to about 20% of the supported catalyst based on metal catalyst weight plus the support weight. A more preferred metal catalyst content range is from about 1% to about 10% of the supported catalyst.

Combinations of metal catalyst and support system may include any one of the metals referred to herein with any of the supports referred to herein. Preferred combinations of metal catalyst and support include palladium on carbon, palladium on alumina, palladium on titania, platinum on carbon, platinum on alumina, platinum on silica, iridium on silica, iridium on carbon, iridium on alumina, rhodium on carbon, rhodium on silica, rhodium on alumina, nickel on carbon, nickel on alumina, nickel on silica, rhenium on carbon, rhenium on silica, rhenium on alumina, ruthenium on carbon, ruthenium on alumina and ruthenium on silica.

Further preferred combinations of metal catalyst and support include ruthenium on carbon, ruthenium on alumina, palladium on carbon, palladium on alumina, palladium on titania, platinum on carbon, platinum on alumina, rhodium on carbon, and rhodium on alumina.

Typically, the hydrogenation reactions are performed at temperatures from about 100°C to about 500°C in reactors maintained at pressures from about 6.89 to about 20.68 MPa.

The method of using the catalyst to hydrogenate a SA or MAS containing feed can be performed by various modes of operation generally known in the art. Thus, the overall hydrogenation process can be performed with a fixed bed reactor, various types of agitated slurry reactors, either gas or mechanically agitated, or the like. The hydrogenation process can be operated in either a batch or continuous mode, wherein an aqueous liquid phase containing the precursor to hydrogenate is in contact with a gaseous phase containing hydrogen at elevated pressure and the particulate solid catalyst.

Temperature, solvent, catalyst, reactor configuration, pressure and mixing rate are all parameters that affect the conversion and selectivity. The relationships among these parameters may be adjusted to effect the desired conversion, reaction rate, and selectivity in the reaction of the process.

A preferred temperature is from about 25°C to 500°C, more preferably from about 100°C to about 400°C, and most preferred from about 150°C to 400°C. The hydrogen pressure is preferably about 0.05 to about 30 MPa.

The processes and/or conversion may be carried out in batch, sequential batch (i.e., a series of batch reactors) or in continuous mode in any of the equipment customarily employed for continuous processes. The condensate water formed as the product of the reaction is removed by separation methods customarily employed for such separations.

### Examples

Our processes are illustrated by the following non-limiting representative examples. In a number of the examples, a synthetic, aqueous DAS solution was used in place of an actual clarified DAS-containing fermentation broth. Other examples use an actual clarified DAS-containing fermentation broth.

The use of a synthetic DAS solution is believed to be a good model for the behavior of an actual broth in our processes because of the solubility of the typical fermentation by-products found in actual broth. The major by-products produced during fermentation are ammonium acetate, ammonium lactate and ammonium formate. If these impurities are present during the distillation step, one would not expect them to lose ammonia and form free acids in significant quantities until all of the DAS had been converted to MAS. This is because acetic acid, lactic acid and formic acid are stronger acids than the second acid group of SA (pKa = 5.48). In other words, acetate, lactate, formate and even monohydrogen succinate are weaker bases than the dianion succinate. Furthermore, ammonium acetate, ammonium lactate and ammonium formate are significantly more soluble in water than MAS, and each is typically present in the broth at less than 10% of the DAS concentration. In addition, even if the acids (acetic, formic and lactic acids) were formed during the distillation step, they are miscible with water and will not crystallize from water. This means that the MAS reaches saturation and crystallizes from solution (i.e., forming the solid portion), leaving the acid impurities dissolved in the mother liquor (i.e., the liquid portion).

### Example 1

This example demonstrates conversion of a portion of DAS into MAS via distillation and recovery of MAS solids from distillation bottoms liquid via cooling-induced crystallization.

A three neck 500 mL round bottom flask was fitted with a thermometer and Dean Stark trap topped with a reflux condenser. The vent from the reflux condenser went to a scrubbing bottle which contained 100g of a 1.4M acetic acid solution. The flask was charged with 400g of a 10% DAS aqueous solution (pH 8.5). The contents of the flask were stirred with a magnetic stirrer and heated with a heating mantle to distill off 320.6g of distillate (an aqueous ammonia solution) which was removed via the Dean Stark trap. Analysis of the distillate indicated that about 20% of the contained ammonia had been removed from the charged DAS during distillation (i.e., the salts in the bottoms liquid were about 40% MAS and about 60% DAS). Only traces of ammonia were found in the scrubbing bottle. The final temperature of the pot as the last drop distilled over was 110°C. The residue (bottoms liquid) in the pot (73.4g which was about 53% water) was placed in a flask and allowed to cool to room temperature overnight. Upon cooling to room temperature, white needles of MAS were formed. The white solids were separated via vacuum filtration, yielding 14g of wet crystals (solid portion) and 56g of mother liquor (liquid portion). A portion of the wet crystals (7g) was dried overnight in a vacuum oven, yielding 6g of dried solids which contained 0.4% water as determined by Karl-Fisher analysis. Analysis of the solids portion with HPLC revealed that the solids portion was free of non-MAS nitrogen-containing impurities (e.g., succinimide and succinamide).

### Example 2

This example demonstrates mother liquor recycle.

A 1-L round bottom flask was charged with 800g of a synthetic 4.5% DAS solution, and then a distillation head was attached to the flask. The contents of the flask were distilled at atmospheric pressure leaving 67g of residue (bottoms liquid) in the flask. The bottoms liquid contained approximately 45% water. Ammonia analyses of the distillates indicate that the first distillation cycle removed about 29% of the ammonia, making a 42/58 mol/mol mixture of DAS and MAS. The residue (bottoms liquid) was then removed from the flask and placed in a beaker equipped with a water bath. The beaker contents were cooled to 20°C with stirring. Once the residue reached 20°C, it was seeded with a few crystals of MAS and allowed to stir for 30 minutes. The temperature of the bath was then lowered to 15°C and held for 30 minutes. The temperature was then lowered to 10°C and held for 30 minutes. The temperature was then cooled to 5°C and held for 30 minutes and finally to 0°C where it was held for 30 minutes. The slurry (consisting of solid and liquid portions) was then quickly filtered using a pre-cooled sintered glass filter funnel and vacuum flask. The solids were dried in a vacuum oven yielding 13.9g of dry MAS solids. The mother liquor (liquid portion, 47.2g) was then combined with 800g of synthetic 4.5% DAS solution and distilled, leaving 86.6g of residue (bottoms liquid). In the second distillation (i.e., mother liquor recycle run) about 28% of the ammonia from the total amount of DAS present was removed. The residue (bottoms liquid) was then cooled (crystallized) in a similar manner. However, the solution became cloudy at 46°C, so it was seeded at 46°C and allowed to slowly cool to room temperature overnight while stirring. The next day the temperature was slowly ramped down by 5°C increments to 0°C. The slurry (solid and liquid portions) was filtered as before, and the solids dried, yielding 23.5g of MAS solids. This is equal to about a 75% recovery of the SA equivalents in the 800g of fresh DAS solution distilled. The recovered solids from the first cycle were 95% MAS (about 5% water). In the second cycle, the solids were 97% MAS (about 3% water). The mother liquor from the second cycle contained 28.8% SA equivalents (i.e., as SA salts).

### Example 3

This example demonstrates the absence of amide and imide species in the solid portion of cooled distillation bottoms.

A 1-L round bottom flask was charged with 800g of a synthetic 4.5% DAS solution. The flask was fitted with a five tray 1" Oldershaw section which was capped with a distillation head. The distillate was collected in an ice cooled receiver. The contents of the flask were heated with a heating mantel and stirred with a magnetic stirrer. The contents of the flask were distilled giving 721.1 g of an overhead distillate and 72.2g of a liquid residue in the flask (i.e. distillation bottoms). The aqueous ammonia distillate was titrated revealing a 0.34% ammonia content (i.e., about 55% conversion of DAS to MAS). The hot distillation bottoms (approximately 47% salt solution of DAS and MAS) were then placed in a 125 mL Erlenmeyer flask and allowed to cool slowly to room temperature while stirring over night. The next morning the cloudy solution was cooled to 15°C and held for 60 minutes, then cooled to 10°C and held for 60 minutes and finally cooled to 5°C and held for 60 minutes while stirring. The resulting white slurry was filtered yielding 12.9g of wet crystals and 55.3g of mother liquor. The crystals were dissolved in 25.8g of distilled water. HPLC analysis of the crystal solution revealed no detectable amounts of amide or imide species. However, HPLC analysis of the mother liquor revealed a trace of succinamic acid, but no detectable succinamide or succinimide.

### Example 4

This example produces a solid portion of a cooled distillation bottoms that consists essentially of MAS and is substantially free of DAS.

A three neck 1-L round bottom flask was fitted with an addition funnel and a 1" five tray Oldershaw column which was capped with a distillation head. An ice cooled receiver was used to collect the distillate. The flask was charged with 800g of a synthetic 4.5% DAS solution. The contents of the flask were heated with a heating mantel and stirred with a magnetic stirrer. Distillation was started. While the distillation occurred an additional 1600g of the 4.5% DAS solution was slowly added to the flask at the same rate as distillate was taken. A total of 2135g of distillate was taken overhead. Titration of the distillate revealed the overhead was a 0.33% ammonia solution. The hot aqueous distillation bottoms (253.8g) was removed from the flask and placed in an Erlenmeyer flask. The distillation bottoms were allowed to slowly cool to room temperature while stirring overnight. The contents of the flask were seeded and allowed to stir for 30 minutes. The slurry was then cooled to 15°C and held for 60 minutes, then 10°C and held for 60 minutes and finally to 5°C and held for 60 minutes all while stirring. The slurry was filtered cold and the solids (i.e., the solid portion) washed three times with about 20g portions of a cold (about 5°C) 20% sodium chloride solution to displace the mother liquor (i.e., the liquid portion). Air was sucked through the cake for several minutes to remove as much liquid as possible. The solids were then dried in a vacuum oven at 75°C for one hour yielding 7.2g of white crystals. Carbon and nitrogen analyses of the solids revealed a 4.06 atomic ratio of carbon to nitrogen (i.e., a 1.01 ratio of ammonia to SA or about 99% MAS). That a ratio of 1.00 was not obtained is believed to be attributable to incomplete washing of the solids.

### Example 5

This example demonstrates the effect of solvents on ammonia evolution from aqueous DAS. Run 5 is the control experiment where no solvent is present.

The outer necks of a three neck 1-L round bottom flask were fitted with a thermometer and a stopper. The center neck was fitted with a five tray 1" Oldershaw section. The Oldershaw section was topped with a distillation head. An ice cooled 500 mL round bottom flask was used as the receiver for the distillation head. The 1-L round bottom flask was charged with distilled water, the solvent being tested, SA and concentrated ammonium hydroxide solution. The contents were stirred with a magnetic stirrer to dissolve all the solids. After the solids dissolved, the contents were heated with the heating mantle to distill 350g of distillate. The distillate was collected in the ice cooled 500 mL round bottom flask. The pot temperature was recorded as the last drop of distillate was collected. The pot contents were allowed to cool to room temperature and the weight of the residue and weight of the distillate were recorded. The ammonia content of the distillate was then determined via titration. The results were recorded in Table 1.

**Table 1**

| Run # | 1 | 2 | 3 | 4 | 5 |
|---|---|---|---|---|---|
| Name of Acid charged | Succinic | Succinic | Succinic | Succinic | Succinic |
| Wt Acid Charged (g) | 11.81 | 11.79 | 11.8 | 11.79 | 11.8 |
| Moles Acid Charged | 0.1 | 0.1 | 0.1 | 0.1 | 0.1 |
| | | | | | |
| Wt 28% NH3 Solution Charged (g) | 12.11 | 12.09 | 12.1 | 12.11 | 12.1 |
| Moles NH3 Charged | 0.2 | 0.2 | 0.2 | 0.2 | 0.2 |
| Name of Solvent | Diglyme | PG* | GBL** | butoxy triglycol | none |
| Wt Solvent Charged (g) | 400 | 400.1 | 400 | 400 | 0 |
| Wt Water Charged (g) | 400 | 400 | 400 | 400 | 800 |
| Wt Distillate (g) | 350.5 | 351.6 | 350.1 | 350.7 | 351 |
| Wt Residue (g) | 466.3 | 461.7 | 464.3 | 460.9 | 466 |
| %Mass Accountability | 99.1 | 98.7 | 98.9 | 98.5 | 99.2 |
| Wt% NH3 in distillate (titration) | 0.48 | 0.4 | 0.27 | 0.47 | 0.13 |
| Moles NH3 in distillate | 0.099 | 0.083 | 0.056 | 0.097 | 0.027 |
| %NH3 removed in Distillate | 49.5 | 42 | 28 | 49 | 13.4 |
| %First NH3 removed in Distillate | 99 | 84 | 56 | 98 | 27 |
| %Second NH3 removed in Distillate | | 0 | 0 | | 0 |
| Final Pot Temp (°C) | 101 | 120 | 110 | 107 | 100 |

| | | | | | |
|---|---|---|---|---|---|
| *PG is propylene glycol ** GBL is gamma butyrolactone | | | | | |

### Example 6

This example produced a solid portion from a cooled distillation bottoms that consists essentially of SA and is substantially free of MAS.

A 300 mL Parr autoclave was charged with 80g of synthetic MAS and 120g of water. The autoclave was sealed and the contents stirred and heated to about 200°C at an autogenic pressure of about 1.31 MPa. Once the contents reached temperature, water was fed to the autoclave at a rate of about 2 g/min and vapor removed from the autoclave at a rate of about 2g/min with a back pressure regulator. Vapor exiting the autoclave was condensed and collected in a receiver. The autoclave was run under these conditions until a total of 1020g of water had been fed and a total of 1019g of distillate collected. The distillate was titrated for ammonia content (0.29% ammonia by weight). This translates into an about 29% conversion of MAS to SA. The contents of the autoclave (194.6g) were partially cooled and discharged from the reactor. The slurry was allowed to stand under stirring at room temperature over night in an Erlenmeyer flask. The slurry was then filtered and the solids rinsed with 25g of water. The moist solids were dried in a vacuum oven at 75°C for 1 hr yielding 9.5g of SA product. Analysis via an ammonium ion electrode revealed 0.013 mmole ammonium ion/g of solid. HPLC analysis revealed the solids were SA with 0.8% succinamic acid impurity.

### Example 7

This example used DAS-containing clarified fermentation broth derived from a fermentation broth containing *E. Coli* strain ATCC PTA-5132. This example produced a solid portion of a cooled distillation bottoms that consists essentially of MAS and is substantially free of DAS.

A three neck 1-L round bottom flask was fitted with an addition funnel and a 1" five tray Oldershaw column which was capped with a distillation head. An ice cooled receiver was used to collect the distillate. The flask was charged with 800g of clarified DAS-containing fermentation broth which contained 4.4% DAS, 1% ammonium acetate, 0.05% ammonium formate and 0.03% ammonium lactate. The contents of the flask were heated with a heating mantel and stirred with a magnetic stirrer. Distillation was started. While the distillation ran, an additional 2200g of the broth solution was slowly added to the flask at the same rate as distillate was removed. A total of 2703g of distillate was taken as overhead. Titration of the distillate revealed the overhead was a 0.28% ammonia solution. The hot aqueous distillation bottoms solution (269.7g) was removed from the flask and placed in an Erlenmeyer flask. The distillation bottoms were allowed to slowly cool to room temperature while stirring overnight. The next day, the contents of the flask were seeded and allowed to stir for 30 minutes. The slurry was then cooled to 15°C and held for 30 minutes, then to 10°C and held for 30 minutes and finally to 5°C and held for 30 minutes, all while stirring. The slurry was filtered cold and air was sucked through the cake for several minutes to remove as much liquid as possible. Light brown solids (72.5g) and dark brown mother liquor (188.4g with a pH of 6.4) were obtained. The solids were recrystallized to remove the mother liquor by dissolution in 72g of water at 50°C. The solution was then allowed to slowly cool to room temperature while stirring overnight. The next day the contents of the flask were seeded and stirred for 30 minutes. The slurry was then cooled to 15°C and held for 30 minutes, then to 10°C and held for 30 minutes, and finally to 5°C and held for 30 minutes, all while stirring. The slurry was filtered cold and air was sucked through the cake for several minutes to remove as much liquid as possible, yielding 110g of brown mother liquor (pH 5.0). The solids were then dried in a vacuum oven at 75°C for one hour yielding 24g of off-white crystals. Carbon and nitrogen analyses of the solids revealed a 4.04 molar ratio of carbon to nitrogen (i.e. a 1.01 ratio of ammonia to SA or about 99% MAS). HPLC analysis revealed that the MAS contained 0.07% succinamic acid but no detectable succinamide, succinimide or acetate species. In other words, the MAS was free of DAS and otherwise substantially pure.

### Example 8

This example used fermentation derived MAS from a fermentation broth containing *E. Coli* strain ATCC PTA-5132. This example produced a solid portion from a cooled distillation bottoms that consists essentially of SA and is substantially free of MAS.

A 300 mL Parr autoclave was charged with 80g of broth derived MAS and 120g of water. The autoclave was sealed and the contents stirred and heated to about 202°C at an autogenic pressure of about 1.41 MPa. Once the contents reached temperature, water was fed to the autoclave at a rate of about 2 g/min and vapor was removed from the autoclave at a rate of about 2g/min with a back pressure regulator. Vapor exiting the autoclave was condensed and collected in a receiver. The autoclave was run under these conditions until a total of 905g of water had been fed and a total of 908g of distillate collected. The distillate was titrated for ammonia content (0.38% ammonia by weight). This translates into an about 34% conversion of MAS to SA. The contents of the autoclave (178.2g) were partially cooled and discharged from the reactor. The slurry was allowed to stand under stirring at room temperature over night in an Erlenmeyer flask. The slurry was then filtered and the solids rinsed with 25g of water. The moist solids were dried in a vacuum oven at 75°C for 1 hr yielding 8.5g of SA product. Analysis via an ammonium ion electrode revealed 0.027 mmole ammonium ion/g of solid. HPLC analysis revealed the solids were SA with 1.4% succinamic acid and 0.1 % succinamide impurities.

### Example 9

This example used an ammonia releasing solvent to aid deammoniation. This example produced a solid portion from a cooled distillation bottoms that consists essentially of SA and is substantially free of MAS.

A 500 mL round bottom flask was charged with 29g of MAS solids, 51 g of water and 80g of triglyme. The flask was fitted with a 5 tray 1" glass Oldershaw column section which was topped with a distillation head. An addition funnel containing 2500g of water was also connected to the flask. The flask was stirred with a magnetic stirrer and heated with a heating mantel. The distillate was collected in an ice cooled receiver. When the distillate started coming over the water in the addition funnel was added to the flask at the same rate as the distillate was being taken. A total of 2491g of distillate was taken. The distillate contained 2.3g of ammonia, as determined by titration. This means about 63% of the MAS was converted to SA. The residue in the flask was then placed in an Erlenmeyer flask and cooled to -5°C while stirring. After stirring for 30 minutes the slurry was filtered while cold yielding 15.3g of solids. The solids were dissolved in 15.3g of hot water and then cooled in an ice bath while stirring. The cold slurry was filtered and the solids dried in a vacuum oven at 100°C for 2 hrs yielding 6.5g of succinic acid. HPLC analysis indicated that the solids were SA with 0.18% succinamic acid present.

### Example 10

This example used an ammonia releasing solvent to aid deammoniation. This example produced a solid portion of a cooled distillation bottoms that consists essentially of MAS and is substantially free of DAS.

A 500 mL round bottom flask was charged with 80g of an aqueous 36% DAS solution and 80g of triglyme. The flask was fitted with a 5 tray 1" glass Oldershaw column section which was topped with a distillation head. An addition funnel containing 700g of water was also connected to the flask. The flask was stirred with a magnetic stirrer and heated with a heating mantel. The distillate was collected in an ice cooled receiver. When the distillate started coming over the water in the addition funnel was added to the flask at the same rate as the distillate was being taken. A total of 747g of distillate was taken. The distillate contained 3.7g of ammonia, as determined by titration. This means about 57% of the ammonia was removed. In other words, all of the DAS was converted into MAS and about 14% of the MAS was further converted into SA. The residue in the flask was then placed in an Erlenmeyer flask and cooled to 5°C while stirring. After stirring for 30 minutes the slurry was filtered while cold and the solids dried in a vacuum oven at 100°C for 2 hrs yielding 10.3g of MAS. Analysis indicated that the solids were MAS with 0.77% succinamic acid and 0.14% succinimide present.

### Example 11

This example demonstrates the use of an azeotroping solvent, particularly separation of MAS from other by-products in the broth.

A three neck 500 mL round bottom flask was fitted with a thermometer, a 250 mL addition funnel and a Dean Stark trap topped with a reflux condenser. The flask was charged with 100g of toluene and 100g of an about 9% DAS broth solution (which also contained about 1% ammonium acetate and ammonium formate combined). The addition funnel was charged with 250g of the 9% diammonim succinate broth solution. The contents of the flask were stirred with a magnetic stirrer and heated with a heating mantel bringing the contents to boil. The contents of the addition funnel were added slowly to the flask allowing the toluene-water azeotope to distill into the Dean-Stark trap with return of the toluene to the flask. After all the contents of the addition funnel had been added (at a rate substantially equal to the distillate) the contents were allowed to further reflux until a total of 277.5g of aqueous phase had been collected from the Dean Stark trap. The contents of the flask were removed while hot and the two phases separated in a warm separatory funnel. The aqueous phase was cooled in an ice bath while being stirred. The resulting solids were recovered via filtration using a sintered glass funnel. The mother liquor was dark brown and the filtered solids were off-white. The solids were dried in a vacuum oven and analyzed via HPLC. The dried solids (5.7g) were about 96% monoammonium succinate and about 1% ammonium acetate with the rest being water.

### Example 12

A pressure distillation column was constructed using an 8 ft long 1.5" 316 SS Schedule 40 pipe packed with 316 SS Propak packing. The base of the column was equipped with an immersion heater to serve as the reboiler. Nitrogen was injected into the reboiler via a needle valve to pressure. The overhead of the column had a total take-off line which went to a 316 SS shell and tube condenser with a receiver. The receiver was equipped with a pressure gauge and a back pressure regulator. Material was removed from the overhead receiver via blowcasing through a needle valve. Preheated feed was injected into the column at the top of the packing via a pump. Preheated water was also injected into the reboiler via a pump. This column was operated at 0.21 MPa pressure which gave a column temperature of 137°C. The top of the column was fed a synthetic 10% DAS solution at a rate of 5 mL/min and water was fed to the reboiler at a rate of 5 mL/min. The overhead distillate rate was 8 mL/min and the residue rate was 2 mL/min. Titration of the distillate for ammonia indicated that the about 47% of the ammonia had been removed in the distillate (i.e. the conversion to MAS was about 94%). The residue liquid was about 20% MAS and HPLC analysis of the residue indicated an about 3% inefficiency to succinamic acid.

### Example 13

A portion of the residue (800g) from Example 12 was concentrated via a batch distillation to about 59% MAS solution (i.e. 530g of water was distilled off). The residue was then cooled to 5°C while stirring. The resulting slurry was filtered and the solids dried in a vacuum oven at 75°C for 1 hour yielding 52.5g of MAS solids (i.e. about 32% recovery). HPLC analysis indicated that the solids contained 0.49% succinamic acid and no succinimide.

### Example 14

A second portion of the pressure column residue (3200g) from Example 12 was placed in the evaporative crystallizer and concentrated to about 72% MAS by distilling off 2312g of water at 60°C under vacuum. The resulting hot slurry was centrifuged and the recovered solids dried in the vacuum oven at 75°C for one hour yielding 130.7g of MAS solids. The mother liquor from the centrifuging step was allowed to cool to room temperature forming a second crop of crystals. This slurry was filtered and the recovered solids were dried at 75°C under vacuum yielding 114.8g of MAS solids. Based on the succinate concentration of the feed to the crystallizer, a 20% and 18% recovery was realized for the first and second crops, respectively (i.e. a 38% overall recovery). HPLC analysis of the two crops of solids indicated that the first crop had no detectable succinamic acid and succinimide while the second crop had 0.96% succinamic acid and 0.28% succinimide.

### Comparative Example 1

This example demonstrates that an atmospheric distillation of an aqueous MAS solution removes very little ammonia when triglyme is not present.

A 500 mL round bottom flask was charged with 30g of MAS solids and 120g of water. The flask was fitted with a 5 tray 1" glass Oldershaw column section which was topped with a distillation head. An addition funnel containing 600g of water was also connected to the flask. The flask was stirred with a magnetic stirrer and heated with a heating mantel. The distillate was collected in an ice cooled receiver. When the distillate started coming over the water in the addition funnel was added to the flask at the same rate as the distillate was being taken. A total of 606g of distillate was taken. The distillate contained 0.15g of ammonia, as determined by titration. This means ∼4% of the MAS was converted to SA.

### Comparative Example 2

This example demonstrates the decrease in ammonia removal for DAS when triglyme is not present.

A 500 mL round bottom flask was charged with 80g of an aqueous 36% DAS solution and 80g of water. The flask was fitted with a 5 tray 1" glass Oldershaw column section which was topped with a distillation head. An addition funnel containing 1200g of water was also connected to the flask. The flask was stirred with a magnetic stirrer and heated with a heating mantel. The distillate was collected in an ice cooled receiver. When the distillate started coming over the water in the addition funnel was added to the flask at the same rate as the distillate was being taken. A total of 1290g of distillate was taken. The distillate contained 2.2g of ammonia, as determined by titration. This means about 44% of the DAS was converted to MAS.

## Claims

1. A process for making nitrogen containing compounds comprising:
(a) providing a clarified diammonium succinate-containing fermentation broth;
(b) distilling the broth to form an overhead that comprises water and ammonia, and a liquid bottoms that comprises monoammonium succinate, at least some diammonium succinate, and at least 20 wt% water;
(c) cooling and/or evaporating the bottoms, and optionally adding an antisolvent to the bottoms, to attain a temperature and composition sufficient to cause the bottoms to separate into a diammonium succinate-containing liquid portion and a monoammonium succinate-containing solid portion that is substantially free of diammonium succinate;
(d) separating at least part of the solid portion from the liquid portion;
(e)
(1) contacting at least a part of the solid portion with hydrogen and, optionally an ammonia source, in the presence of a hydrogenation catalyst at a temperature of 150°C to 400°C and a pressure of 0.68 to 27.6 MPa to produce the compound of Formula I; or
(2) contacting at least a part of the solid portion with hydrogen and either an alkylamine of the formula R-NH₂ or an alcohol of the formula R-OH, wherein R is a linear or branched C₁ to C₂₀ alkyl group or a C₅ to C₂₀ substituted or unsubstituted cycloalkyl group or an aromatic group C₆ or larger, and, optionally an ammonia source, in the presence of a hydrogenation catalyst at a temperature of 150°C to 400°C and a pressure of 0.68 to 27.6 MPa to produce the compound of Formula II; or
(3) contacting at least a part of the solid portion with hydrogen and NH2CH2CH2OH or ethylene glycol and hydrogen and, optionally an ammonia source, in the presence of a hydrogenation catalyst at a temperature of 150°C to 400°C and a pressure of 0.68 to 27.6 MPa to produce the compound of Formula III; and
(f) recovering the compounds of Formula I, Formula II or Formula III

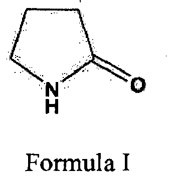

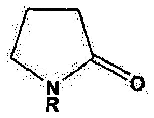

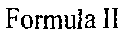

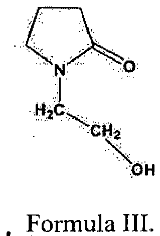

2. A process according to claim 1 for making nitrogen containing compounds comprising:
(a) providing a clarified diammonium succinate -containing fermentation broth;
(b) distilling the broth to form a first overhead that includes water and ammonia, and a first liquid bottoms that includes monoammonium succinate, at least some diammonium succinate, and at least 20 wt% water;
(c) cooling and/or evaporating the bottoms, and optionally adding an antisolvent to the bottoms, to attain a temperature and composition sufficient to cause the bottoms to separate into a diammonium succinate-containing liquid portion and a monoammonium succinate-containing solid portion that is substantially free of diammonium succinate;
(d) separating the solid portion from the liquid portion;
(e) recovering the solid portion;
(f) dissolving the solid portion in water to produce an aqueous monoammonium succinate solution;
(g) distilling the aqueous monoammonium succinate solution at a temperature and pressure sufficient to form a second overhead that includes water and ammonia, and a second bottoms that includes a major portion of succinic acid, a minor portion of monoammonium succinate, and water;
(h) cooling and/or evaporating the second bottoms to cause the second bottoms to separate into a second liquid portion in contact with a second solid portion that preferably consists essentially of succinic acid and is substantially free of monoammonium succinate;
(i) separating at least part of the second solid portion from the second liquid portion;
(j)
(1) contacting at least a part of the solid portion with hydrogen and, optionally an ammonia source, in the presence of a hydrogenation catalyst at a temperature of 150°C to 400°C and a pressure of 0.68 to 27.6 MPa to produce the compound of Formula I; or
(2) contacting at least a part of the solid portion with hydrogen and either an alkylamine of the formula R-NH₂ or an alcohol of the formula R-OH, wherein R is a linear or branched C₁ to C₂₀ alkyl group or a C₅ to C₂₀ substituted or unsubstituted cycloalkyl group or an aromatic group C₆ or larger, and, optionally an ammonia source, in the presence of a hydrogenation catalyst at a temperature of 150°C to 400°C and a pressure of 0.68 to 27.6 MPa to produce the compound of Formula II; or
(3) contacting at least a part of the solid portion with hydrogen and NH₂CH₂CH₂OH or ethylene glycol and hydrogen and, optionally an ammonia source, in the presence of a hydrogenation catalyst at a temperature of 150°C to 400°C and a pressure of 0.68 to 27.6 MPa to produce the compound of Formula III; and
(k) recovering the compounds of Formula I, Formula II or Formula III

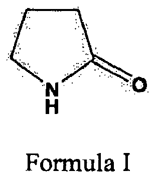

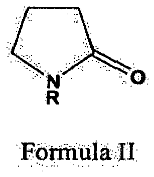

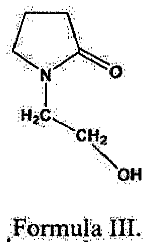

3. A process according to claim 1 for making nitrogen containing compounds comprising:
(a) providing a clarified monoammonium succinate-containing fermentation broth;
(b) optionally, adding monoammonium, succinate, diammonium succinate, succinic acid, NH₃, and/or NH₄⁺ to the broth to preferably maintain the pH of the broth below 6;
(c) distilling the broth to form an overhead that includes water and optionally ammonia, and a liquid bottoms that includes monoammonium succinate, at least some diammonium succinate, and at least 20 wt% water;
(d) cooling and/or evaporating the bottoms, and optionally adding an antisolvent to the bottoms, to attain a temperature and composition sufficient to cause the bottoms to separate into a diammonium succinate-containing liquid portion and a monoammonium succinate-containing solid portion that is substantially free of diammonium succinate;
(e) separating at least part of the solid portion from the liquid portion;
(f)
(1) contacting at least a part of the solid portion with hydrogen and, optionally an ammonia source, in the presence of a hydrogenation catalyst at a temperature of 150°C to 400°C and a pressure of 0.68 to 27.6 MPa to produce the compound of Formula I; or
(2) contacting at least a part of the solid portion with hydrogen and either an alkylamine of the formula R-NH₂ or an alcohol of the formula R-OH, wherein R is a linear or branched C₁ to C₂₀ alkyl group or a C₅ to C₂₀ substituted or unsubstituted cycloalkyl group or an aromatic group C₆ or larger, and, optionally an ammonia source, in the presence of a hydrogenation catalyst at a temperature of 150°C to 400°C and a pressure of 0.68 to 27.6 MPa to produce the compound of Formula II; or
(3) contacting at least a part of the solid portion with hydrogen and NH₂CH₂CH₂OH or ethylene glycol and hydrogen and, optionally an ammonia source, in the presence of a hydrogenation catalyst at a temperature of 150°C to 400°C and a pressure of 0.68 to 27.6 MPa to produce the compound of Formula III; and
(g) recovering the compounds of Formula I, Formula II or Formula III

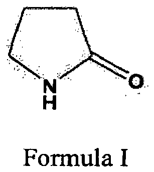

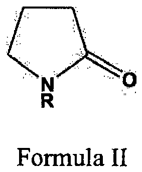

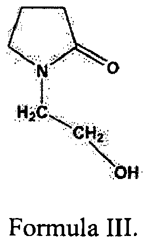

4. A process according to claim 1 for making nitrogen containing compounds comprising:
(a) providing a clarified monoammonium succinate-containing fermentation broth;
(b) optionally, adding monoammonium succinate, diammonium succinate, succinic acid, NH₃, and/or NH₄⁺ to the broth to preferably maintain the pH of the broth below 6;
(c) distilling the broth to form an overhead that includes water and optionally ammonia, and a liquid bottoms that includes monoammonium succinate, at least some diammonium succinate, and at least 20 wt% water;
(d) cooling and/or evaporating the bottoms, and optionally adding an antisolvent to the bottoms, to attain a temperature and composition sufficient to cause the bottoms to separate into a diammonium succinate-containing liquid portion and a monoammonium succinate-containing solid portion that is substantially free of diammonium succinate;
(e) separating the solid portion from the liquid portion; and
(f) recovering the solid portion;
(g) dissolving the solid portion in water to produce an aqueous monoammonium succinate solution;
(h) distilling the aqueous monoammonium succinate solution at a temperature and pressure sufficient to form a second overhead that includes water and ammonia, and a second bottoms that includes a major portion of succinic acid, a minor portion of monoammonium succinate, and water;
(i) cooling and/or evaporating the second bottoms to cause the second bottoms to separate into a second liquid portion in contact with a second solid portion that preferably consists essentially of succinic acid and is substantially free of monoammonium succinate;
(j) separating at least part of the second solid portion from the second liquid portion;
(k)
(1) contacting at least a part of the solid portion with hydrogen and, optionally an ammonia source, in the presence of a hydrogenation catalyst at a temperature of 150°C to 400°C and a pressure of 0.68 to 27.6 MPa to produce the compound of Formula I; or
(2) contacting at least a part of the solid portion with hydrogen and either an alkylamine of the formula R-NH₂ or an alcohol of the formula R-OH, wherein R is a linear or branched C₁ to C₂₀ alkyl group or a C₅ to C₂₀ substituted or unsubstituted cycloalkyl group or an aromatic group C₆ or larger, and, optionally an ammonia source, in the presence of a hydrogenation catalyst at a temperature of 150°C to 400°C and a pressure of 0.68 to 27.6 MPa to produce the compound of Formula II; or
(3) contacting at least a part of the solid portion with hydrogen and NH₂CH₂CH₂OH or ethylene glycol and hydrogen and, optionally an ammonia source, in the presence of a hydrogenation catalyst at a temperature of 150°C to 400°C and a pressure of 0.68 to 27.6 MPa to produce the compound of Formula III; and
(l) recovering the compounds of Formula I, Formula II or Formula III

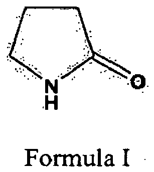

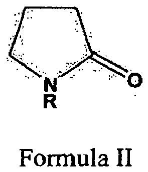

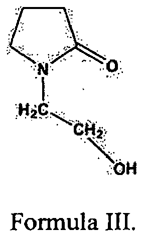

5. The processes of any of claims 1-4, further comprising contacting the compound of Formula I with acetylene in the presence of a basic catalyst at a temperature of 80°C to 250°C and a pressure of 0.5 to 25 MPa to produce the compound of Formula IV

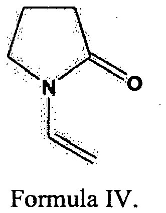

6. The processes of any of claims 1-4, further comprising dehydrating the compound of Formula III at a temperature of 100°C to 500°C and a pressure of 0.068 to 1.37 MPa to produce the compound of Formula IV

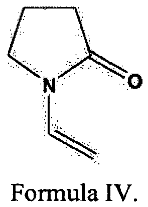

7. The processes of any of claims 1-4, wherein the distillations are carried out in the presence of an ammonia separating solvent which is at least one selected from the group consisting of diglyme, triglyme, tetraglyme, sulfoxides, amides, sulfones, polyethyleneglycol (PEG), butoxytriglycol, N-methylpyrolidone (NMP), ethers, and methyl ethyl ketone (MEK) or in the presence of a water azeotroping solvent which is at least one selected from the group consisting of toluene, xylene, methylcyclohexane, methyl isobutyl ketone, hexane, cyclohexane and heptane.

## Patentansprüche

1. Verfahren zur Herstellung Stickstoff enthaltender Verbindungen, umfassend:
(a) Bereitstellen einer geklärten Fermentationsbrühe, die Diammoniumsuccinat enthält,
(b) Destillieren der Brühe, so dass ein Vorlauf gebildet wird, der Wasser und Ammoniak umfasst, und ein flüssiger Rückstand gebildet wird, der Monoammoniumsuccinat, mindestens etwas Diammoniumsuccinat und mindestens 20 Gew.-% Wasser umfasst,
(c) Abkühlen und/oder Verdampfen des Rückstands und optional Zugeben eines Nichtlösungsmittels zum Rückstand, um eine Temperatur und Zusammensetzung zu erreichen, die ausreichend sind, um eine Trennung des Rückstands in einen Diammoniumsuccinat enthaltenden Flüssigkeitsanteil und einen Monoammoniumsuccinat enthaltenden Feststoffanteil, der im Wesentlichen von Diammoniumsuccinat frei ist, zu bewirken,
(d) Abtrennen mindestens eines Teils des Feststoffanteils vom Flüssigkeitsanteil,
(e)
(1) in Kontakt bringen mindestens eines Teils des Feststoffanteils mit Wasserstoff, und optional einer Ammoniakquelle, in Gegenwart eines Hydrierungskatalysators bei einer Temperatur von 150 °C bis 400 °C und einem Druck von 0,68 bis 27,6 MPa, so dass die Verbindung der Formel I entsteht, oder
(2) in Kontakt bringen mindestens eines Teils des Feststoffanteils mit Wasserstoff und entweder einem Alkylamin der Formel R-NH₂ oder einem Alkohol der Formel R-OH, worin R eine lineare oder verzweigte C₁- bis C₂₀-Alkylgruppe oder eine substituierte oder unsubstituierte C₅- bis C₂₀-Cycloalkylgruppe oder eine aromatische Gruppe mit C₆ oder größer ist, und optional einer Ammoniakquelle, in Gegenwart eines Hydrierungskatalysators bei einer Temperatur von 150 °C bis 400 °C und einem Druck von 0,68 bis 27,6 MPa, so dass die Verbindung der Formel II entsteht, oder
(3) in Kontakt bringen mindestens eines Teils des Feststoffanteils mit Wasserstoff und NH₂CH₂CH₂OH oder Ethylenglycol und Wasserstoff, und optional einer Ammoniakquelle, in Gegenwart eines Hydrierungskatalysators bei einer Temperatur von 150 °C bis 400 °C und einem Druck von 0,68 bis 27,6 MPa, so dass die Verbindung der Formel III entsteht, und
(f) Gewinnen der Verbindungen der Formel I, Formel II oder Formel III

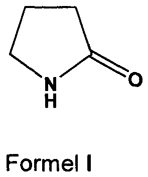

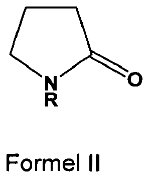

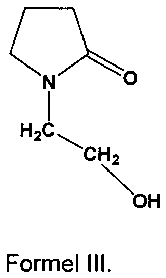

2. Verfahren nach Anspruch 1 zur Herstellung Stickstoff enthaltender Verbindungen, umfassend:
(a) Bereitstellen einer geklärten Fermentationsbrühe, die Diammoniumsuccinat enthält,
(b) Destillieren der Brühe, so dass ein erster Vorlauf gebildet wird, der Wasser und Ammoniak beinhaltet, und ein erster flüssiger Rückstand gebildet wird, der Monoammoniumsuccinat, mindestens etwas Diammoniumsuccinat und mindestens 20 Gew.-% Wasser beinhaltet,
(c) Abkühlen und/oder Verdampfen des Rückstands und optional Zugeben eines Nichtlösungsmittels zum Rückstand, um eine Temperatur und Zusammensetzung zu erreichen, die ausreichend sind, um eine Trennung des Rückstands in einen Diammoniumsuccinat enthaltenden Flüssigkeitsanteil und einen Monoammoniumsuccinat enthaltenden Feststoffanteil, der im Wesentlichen von Diammoniumsuccinat frei ist, zu bewirken,
(d) Abtrennen des Feststoffanteils vom Flüssigkeitsanteil,
(e) Gewinnen des Feststoffanteils,
(f) Auflösen des Feststoffanteils in Wasser, so dass eine wässrige Monoammoniumsuccinatlösung gebildet wird,
(g) Destillieren der wässrigen Monoammoniumsuccinatlösung bei einer Temperatur und einem Druck, die ausreichend sind, um einen zweiten Vorlauf zu bilden, der Wasser und Ammoniak beinhaltet, und einen zweiten Rückstand zu bilden, der einen größeren Anteil an Bernsteinsäure, einen kleineren Anteil an Monoammoniumsuccinat und Wasser beinhaltet,
(h) Abkühlen und/oder Verdampfen des zweiten Rückstands, um eine Trennung des zweiten Rückstands in einen zweiten Flüssigkeitsanteil in Kontakt mit einem zweiten Feststoffanteil, der bevorzugt im Wesentlichen aus Bernsteinsäure besteht und im Wesentlichen von Monoammoniumsuccinat frei ist, zu bewirken,
(i) Abtrennen mindestens eines Teils des zweiten Feststoffanteils vom zweiten Flüssigkeitsanteil,
(j)
(1) in Kontakt bringen mindestens eines Teils des Feststoffanteils mit Wasserstoff, und optional einer Ammoniakquelle, in Gegenwart eines Hydrierungskatalysators bei einer Temperatur von 150 °C bis 400 °C und einem Druck von 0,68 bis 27,6 MPa, so dass die Verbindung der Formel I entsteht, oder
(2) in Kontakt bringen mindestens eines Teils des Feststoffanteils mit Wasserstoff und entweder einem Alkylamin der Formel R-NH₂ oder einem Alkohol der Formel R-OH, worin R eine lineare oder verzweigte C₁- bis C₂₀-Alkylgruppe oder eine substituierte oder unsubstituierte C₅- bis C₂₀-Cycloalkylgruppe oder eine aromatische Gruppe mit C₆ oder größer ist, und optional einer Ammoniakquelle, in Gegenwart eines Hydrierungskatalysators bei einer Temperatur von 150 °C bis 400 °C und einem Druck von 0,68 bis 27,6 MPa, so dass die Verbindung der Formel II entsteht, oder
(3) in Kontakt bringen mindestens eines Teils des Feststoffanteils mit Wasserstoff und NH₂CH₂CH₂OH oder Ethylenglycol und Wasserstoff, und optional einer Ammoniakquelle, in Gegenwart eines Hydrierungskatalysators bei einer Temperatur von 150 °C bis 400 °C und einem Druck von 0,68 bis 27,6 MPa, so dass die Verbindung der Formel III entsteht, und
(k) Gewinnen der Verbindungen der Formel I, Formel II oder Formel III

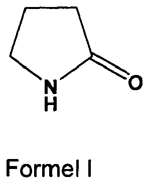

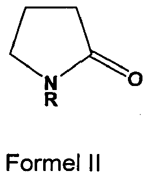

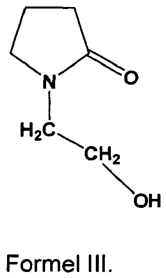

3. Verfahren nach Anspruch 1 zur Herstellung Stickstoff enthaltender Verbindungen, umfassend:
(a) Bereitstellen einer geklärten Fermentationsbrühe, die Monoammoniumsuccinat enthält,
(b) optional Zugeben von Monoammoniumsuccinat, Diammoniumsuccinat, Bernsteinsäure, NH₃ und/oder NH₄⁺ zur Brühe, so dass der pH der Brühe bevorzugt unter 6 gehalten wird,
(c) Destillieren der Brühe, so dass ein Vorlauf gebildet wird, der Wasser und optional Ammoniak beinhaltet, und ein flüssiger Rückstand gebildet wird, der Monoammoniumsuccinat, mindestens etwas Diammoniumsuccinat und mindestens 20 Gew.-% Wasser beinhaltet,
(d) Abkühlen und/oder Verdampfen des Rückstands und optional Zugeben eines Nichtlösungsmittels zum Rückstand, um eine Temperatur und Zusammensetzung zu erreichen, die ausreichend sind, um eine Trennung des Rückstands in einen Diammoniumsuccinat enthaltenden Flüssigkeitsanteil und einen Monoammoniumsuccinat enthaltenden Feststoffanteil, der im Wesentlichen von Diammoniumsuccinat frei ist, zu bewirken,
(e) Abtrennen mindestens eines Teils des Feststoffanteils vom Flüssigkeitsanteil,
(f)
(1) in Kontakt bringen mindestens eines Teils des Feststoffanteils mit Wasserstoff, und optional einer Ammoniakquelle, in Gegenwart eines Hydrierungskatalysators bei einer Temperatur von 150 °C bis 400 °C und einem Druck von 0,68 bis 27,6 MPa, so dass die Verbindung der Formel I entsteht, oder
(2) in Kontakt bringen mindestens eines Teils des Feststoffanteils mit Wasserstoff und entweder einem Alkylamin der Formel R-NH₂ oder einem Alkohol der Formel R-OH, worin R eine lineare oder verzweigte C₁- bis C₂₀-Alkylgruppe oder eine substituierte oder unsubstituierte C₅- bis C₂₀-Cycloalkylgruppe oder eine aromatische Gruppe mit C₆ oder größer ist, und optional einer Ammoniakquelle, in Gegenwart eines Hydrierungskatalysators bei einer Temperatur von 150 °C bis 400 °C und einem Druck von 0,68 bis 27,6 MPa, so dass die Verbindung der Formel II entsteht, oder
(3) in Kontakt bringen mindestens eines Teils des Feststoffanteils mit Wasserstoff und NH₂CH₂CH₂OH oder Ethylenglycol und Wasserstoff, und optional einer Ammoniakquelle, in Gegenwart eines Hydrierungskatalysators bei einer Temperatur von 150 °C bis 400 °C und einem Druck von 0,68 bis 27,6 MPa, so dass die Verbindung der Formel III entsteht, und
(g) Gewinnen der Verbindungen der Formel I, Formel II oder Formel III

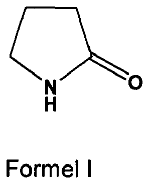

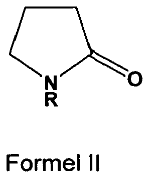

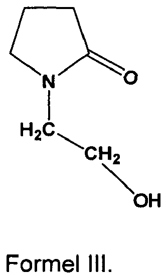

4. Verfahren nach Anspruch 1 zur Herstellung Stickstoff enthaltender Verbindungen, umfassend:
(a) Bereitstellen einer geklärten Fermentationsbrühe, die Monoammoniumsuccinat enthält,
(b) optional Zugeben von Monoammoniumsuccinat, Diammoniumsuccinat, Bernsteinsäure, NH₃ und/oder NH₄⁺ zur Brühe, so dass der pH der Brühe bevorzugt unter 6 gehalten wird,
(c) Destillieren der Brühe, so dass ein Vorlauf gebildet wird, der Wasser und optional Ammoniak beinhaltet, und ein flüssiger Rückstand gebildet wird, der Monoammoniumsuccinat, mindestens etwas Diammoniumsuccinat und mindestens 20 Gew.-% Wasser beinhaltet,
(d) Abkühlen und/oder Verdampfen des Rückstands und optional Zugeben eines Nichtlösungsmittels zum Rückstand, um eine Temperatur und Zusammensetzung zu erreichen, die ausreichend sind, um eine Trennung des Rückstands in einen Diammoniumsuccinat enthaltenden Flüssigkeitsanteil und einen Monoammoniumsuccinat enthaltenden Feststoffanteil, der im Wesentlichen von Diammoniumsuccinat frei ist, zu bewirken,
(e) Abtrennen des Feststoffanteils vom Flüssigkeitsanteil und
(f) Gewinnen des Feststoffanteils,
(g) Auflösen des Feststoffanteils in Wasser, so dass eine wässrige Monoammoniumsuccinatlösung gebildet wird,
(h) Destillieren der wässrigen Monoammoniumsuccinatlösung bei einer Temperatur und einem Druck, die ausreichend sind, um einen zweiten Vorlauf zu bilden, der Wasser und Ammoniak beinhaltet, und einen zweiten Rückstand zu bilden, der einen größeren Anteil an Bernsteinsäure, einen kleineren Anteil an Monoammoniumsuccinat und Wasser beinhaltet,
(i) Abkühlen und/oder Verdampfen des zweiten Rückstands, um eine Trennung des zweiten Rückstands in einen zweiten Flüssigkeitsanteil in Kontakt mit einem zweiten Feststoffanteil, der bevorzugt im Wesentlichen aus Bernsteinsäure besteht und im Wesentlichen von Monoammoniumsuccinat frei ist, zu bewirken,
(j) Abtrennen mindestens eines Teils des zweiten Feststoffanteils vom zweiten Flüssigkeitsanteil,
(k)
(1) in Kontakt bringen mindestens eines Teils des Feststoffanteils mit Wasserstoff, und optional einer Ammoniakquelle, in Gegenwart eines Hydrierungskatalysators bei einer Temperatur von 150 °C bis 400 °C und einem Druck von 0,68 bis 27,6 MPa, so dass die Verbindung der Formel I entsteht, oder
(2) in Kontakt bringen mindestens eines Teils des Feststoffanteils mit Wasserstoff und entweder einem Alkylamin der Formel R-NH₂ oder einem Alkohol der Formel R-OH, worin R eine lineare oder verzweigte C₁- bis C₂₀-Alkylgruppe oder eine substituierte oder unsubstituierte C₅- bis C₂₀-Cycloalkylgruppe oder eine aromatische Gruppe mit C₆ oder größer ist, und optional einer Ammoniakquelle, in Gegenwart eines Hydrierungskatalysators bei einer Temperatur von 150 °C bis 400 °C und einem Druck von 0,68 bis 27,6 MPa, so dass die Verbindung der Formel II entsteht, oder
(3) in Kontakt bringen mindestens eines Teils des Feststoffanteils mit Wasserstoff und NH₂CH₂CH₂OH oder Ethylenglycol und Wasserstoff, und optional einer Ammoniakquelle, in Gegenwart eines Hydrierungskatalysators bei einer Temperatur von 150 °C bis 400 °C und einem Druck von 0,68 bis 27,6 MPa, so dass die Verbindung der Formel III entsteht, und
(l) Gewinnen der Verbindungen der Formel I, Formel II oder Formel III

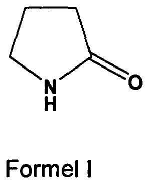

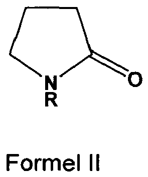

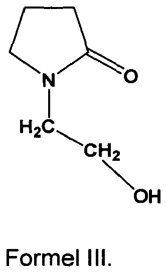

5. Verfahren nach einem der Ansprüche 1 bis 4, weiter umfassend in Kontakt bringen der Verbindung der Formel I mit Acetylen in Gegenwart eines basischen Katalysators bei einer Temperatur von 80 °C bis 250 °C und einem Druck von 0,5 bis 25 MPa, so dass die Verbindung der Formel IV entsteht

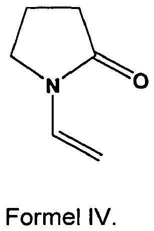

6. Verfahren nach einem der Ansprüche 1 bis 4, weiter umfassend Dehydratisieren der Verbindung der Formel III bei einer Temperatur von 100 °C bis 500 °C und einem Druck von 0,068 bis 1,37 MPa, so dass die Verbindung der Formel IV entsteht

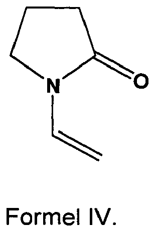

7. Verfahren nach einem der Ansprüche 1 bis 4, worin die Destillationen in Gegenwart eines Ammoniak abtrennenden Lösungsmittels ausgeführt werden, wobei das Lösungsmittel mindestens eines ausgewählt aus der Gruppe bestehend aus Diglyme, Triglyme, Tetraglyme, Sulfoxiden, Amiden, Sulfonen, Polyethylenglycol (PEG), Butoxytriglycol, N-Methylpyrrolidon (NMP), Ethern und Methylethylketon (MEK) ist oder in Gegenwart eines mit Wasser ein Azeotrop bildendes Lösungsmittels ausgeführt werden, wobei das Lösungsmittel mindestens eines ausgewählt aus der Gruppe bestehend aus Toluol, Xylol, Methylcyclohexan, Methylisobutylketon, Hexan, Cyclohexan und Heptan ist.

## Revendications

1. Procédé de fabrication de composés contenant de l'azote comprenant les étapes consistant à :
(a) se procurer un bouillon de fermentation clarifié contenant du succinate de diammonium ;
(b) distiller le bouillon pour former un produit de tête qui comprend de l'eau et de l'ammoniac, et un produit de queue liquide qui comprend du succinate de monoammonium, au moins un peu de succinate de diammonium, et au moins 20 % en poids d'eau ;
(c) refroidir et/ou évaporer le produit de queue, et éventuellement ajouter un anti-solvant au produit de queue, pour atteindre une température et une composition suffisantes pour que le produit de queue se sépare en une fraction liquide contenant du succinate de diammonium et une fraction solide contenant du succinate de monoammonium qui ne contient pratiquement pas de succinate de diammonium ;
(d) séparer de la fraction liquide au moins une partie de la fraction solide ;
(e)
(1) mettre en contact au moins une partie de la fraction solide avec de l'hydrogène et éventuellement une source d'ammoniac, en présence d'un catalyseur d'hydrogénation à une température de 150°C à 400°C et une pression de 0,68 à 27,6 MPa, pour produire le composé de Formule I ; ou
(2) mettre en contact au moins une partie de la fraction solide avec de l'hydrogène et soit une alkyl-amine de formule R-NH₂ soit un alcool de formule R-OH, où R représente un groupe alkyle en C₁ à C₂₀ linéaire ou ramifié ou un groupe cycloalkyle substitué ou non substitué en C₅ à C₂₀ ou un groupe aromatique en C₆ ou plus grand, et éventuellement une source d'ammoniac, en présence d'un catalyseur d'hydrogénation à une température de 150°C à 400°C et une pression de 0,68 à 27,6 MPa, pour produire le composé de Formule II ; ou
(3) mettre en contact au moins une partie de la fraction solide avec de l'hydrogène et NH₂CH₂CH₂OH ou de l'éthylèneglycol et de l'hydrogène, et éventuellement une source d'ammoniac, en présence d'un catalyseur d'hydrogénation à une température de 150°C à 400°C et une pression de 0,68 à 27,6 MPa, pour produire le composé de Formule III ; et
(f) récupérer les composés de Formule I, de Formule II ou de Formule III

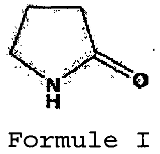

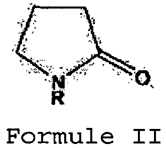

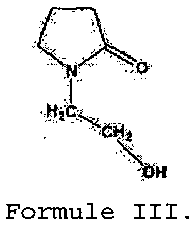

2. Procédé selon la revendication 1 de fabrication de composés contenant de l'azote comprenant les étapes consistant à :
(a) se procurer un bouillon de fermentation clarifié contenant du succinate de diammonium ;
(b) distiller le bouillon pour former un premier produit de tête qui contient de l'eau et de l'ammoniac, et un premier produit de queue liquide qui contient du succinate de monoammonium, au moins un peu de succinate de diammonium, et au moins 20 % en poids d'eau ;
(c) refroidir et/ou évaporer le produit de queue, et éventuellement ajouter un anti-solvant au produit de queue, pour atteindre une température et une composition suffisantes pour que le produit de queue se sépare en une fraction liquide contenant du succinate de diammonium et une fraction solide contenant du succinate de monoammonium qui ne contient pratiquement pas de succinate de diammonium ;
(d) séparer la fraction solide de la fraction liquide ;
(e) récupérer la fraction solide ;
(f) dissoudre la fraction solide dans de l'eau pour produire une solution aqueuse de succinate de monoammonium ;
(g) distiller la solution aqueuse de succinate de monoammonium à une température et une pression suffisantes pour former un second produit de tête qui contient de l'eau et de l'ammoniac, et un second produit de queue qui contient une majorité d'acide succinique, une minorité de succinate de monoammonium, et de l'eau ;
(h) refroidir et/ou évaporer le second produit de queue pour que le second produit de queue se sépare en une seconde fraction liquide en contact avec une seconde fraction solide qui est de préférence constituée essentiellement d'acide succinique et ne contient pratiquement pas de succinate de monoammonium ;
(i) séparer de la seconde fraction liquide au moins une partie de la seconde fraction solide ;
(j)
(1) mettre en contact au moins une partie de la fraction solide avec de l'hydrogène et éventuellement une source d'ammoniac, en présence d'un catalyseur d'hydrogénation à une température de 150°C à 400°C et une pression de 0,68 à 27,6 MPa, pour produire le composé de Formule I ; ou
(2) mettre en contact au moins une partie de la fraction solide avec de l'hydrogène et soit une alkyl-amine de formule R-NH₂ soit un alcool de formule R-OH, où R représente un groupe alkyle en C₁ à C₂₀ linéaire ou ramifié ou un groupe cycloalkyle substitué ou non substitué en C₅ à C₂₀ ou un groupe aromatique en C₆ ou plus grand, et éventuellement une source d'ammoniac, en présence d'un catalyseur d'hydrogénation à une température de 150°C à 400°C et une pression de 0,68 à 27,6 MPa, pour produire le composé de Formule II ; ou
(3) mettre en contact au moins une partie de la fraction solide avec de l'hydrogène et NH₂CH₂CH₂OH ou de l'éthylèneglycol et de l'hydrogène, et éventuellement une source d'ammoniac, en présence d'un catalyseur d'hydrogénation à une température de 150°C à 400°C et une pression de 0,68 à 27,6 MPa, pour produire le composé de Formule III ; et
(k) récupérer les composés de Formule I, de Formule II ou de Formule III

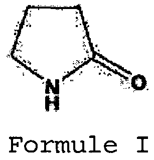

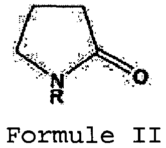

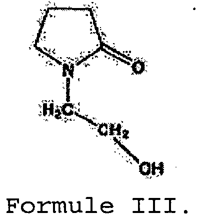

3. Procédé selon la revendication 1 de fabrication de composés contenant de l'azote comprenant les étapes consistant à :
(a) se procurer un bouillon de fermentation clarifié contenant du succinate de monoammonium ;
(b) éventuellement, ajouter au bouillon du succinate de monoammonium, du succinate de diammonium, de l'acide succinique, NH₃ et/ou NH₄⁺ pour maintenir de préférence le pH du bouillon au-dessous de 6 ;
(c) distiller le bouillon pour former un produit de tête qui contient de l'eau et éventuellement de l'ammoniac, et un produit de queue liquide qui contient du succinate de monoammonium, au moins un peu de succinate de diammonium, et au moins 20 % en poids d'eau ;
(d) refroidir et/ou évaporer le produit de queue, et éventuellement ajouter un anti-solvant au produit de queue, pour atteindre une température et une composition suffisantes pour que le produit de queue se sépare en une fraction liquide contenant du succinate de diammonium et une fraction solide contenant du succinate de monoammonium qui ne contient pratiquement pas de succinate de diammonium ;
(e) séparer de la fraction liquide au moins une partie de la fraction solide ;
(f)
(1) mettre en contact au moins une partie de la fraction solide avec de l'hydrogène et éventuellement une source d'ammoniac, en présence d'un catalyseur d'hydrogénation à une température de 150°C à 400°C et une pression de 0,68 à 27,6 MPa, pour produire le composé de Formule I ; ou
(2) mettre en contact au moins une partie de la fraction solide avec de l'hydrogène et soit une alkyl-amine de formule R-NH₂ soit un alcool de formule R-OH, où R représente un groupe alkyle en C₁ à C₂₀ linéaire ou ramifié ou un groupe cycloalkyle substitué ou non substitué en C₅ à C₂₀ ou un groupe aromatique en C₆ ou plus grand, et éventuellement une source d'ammoniac, en présence d'un catalyseur d'hydrogénation à une température de 150°C à 400°C et une pression de 0,68 à 27,6 MPa, pour produire le composé de Formule II ; ou
(3) mettre en contact au moins une partie de la fraction solide avec de l'hydrogène et NH₂CH₂CH₂OH ou de l'éthylèneglycol et de l'hydrogène, et éventuellement une source d'ammoniac, en présence d'un catalyseur d'hydrogénation à une température de 150°C à 400°C et une pression de 0,68 à 27,6 MPa, pour produire le composé de Formule III ; et
(g) récupérer les composés de Formule I, de Formule II ou de Formule III

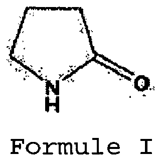

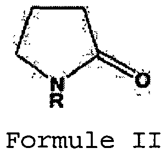

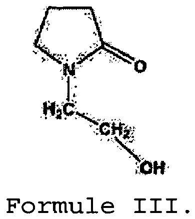

4. Procédé selon la revendication 1 de fabrication de composés contenant de l'azote comprenant les étapes consistant à :
(a) se procurer un bouillon de fermentation clarifié contenant du succinate de monoammonium ;
(b) éventuellement, ajouter du succinate de monoammonium, du succinate de diammonium, de l'acide succinique, NH₃ et/ou NH₄⁺ au bouillon pour maintenir de préférence le pH du bouillon au-dessous de 6 ;
(c) distiller le bouillon pour former un produit de tête qui contient de l'eau et éventuellement de l'ammoniac, et un produit de queue liquide qui contient du succinate de monoammonium, au moins un peu de succinate de diammonium, et au moins 20 % en poids d'eau ;
(d) refroidir et/ou évaporer le produit de queue, et éventuellement ajouter un anti-solvant au produit de queue, pour atteindre une température et une composition suffisantes pour que le produit de queue se sépare en une fraction liquide contenant du succinate de diammonium et une fraction solide contenant du succinate de monoammonium qui ne contient pratiquement pas de succinate de diammonium ;
(e) séparer la fraction solide de la fraction liquide ; et
(f) récupérer la fraction solide ;
(g) dissoudre la fraction solide dans de l'eau pour produire une solution aqueuse de succinate de monoammonium ;
(h) distiller la solution aqueuse de succinate de monoammonium à une température et une pression suffisantes pour former un second produit de tête qui contient de l'eau et de l'ammoniac, et un second produit de queue qui contient une majorité d'acide succinique, une minorité de succinate de monoammonium, et de l'eau ;
(i) refroidir et/ou évaporer le second produit de queue pour que le second produit de queue se sépare en une seconde fraction liquide en contact avec une seconde fraction solide qui est de préférence constituée essentiellement d'acide succinique et ne contient pratiquement pas de succinate de monoammonium ;
(j) séparer de la seconde fraction liquide au moins une partie de la seconde fraction solide ;
(k)
(1) mettre en contact au moins une partie de la fraction solide avec de l'hydrogène et éventuellement une source d'ammoniac, en présence d'un catalyseur d'hydrogénation à une température de 150°C à 400°C et une pression de 0,68 à 27,6 MPa, pour produire le composé de Formule I ; ou
(2) mettre en contact au moins une partie de la fraction solide avec de l'hydrogène et soit une alkyl-amine de formule R-NH₂ soit un alcool de formule R-OH, où R représente un groupe alkyle en C₁ à C₂₀ linéaire ou ramifié ou un groupe cycloalkyle substitué ou non substitué en C₅ à C₂₀ ou un groupe aromatique en C₆ ou plus grand, et éventuellement une source d'ammoniac, en présence d'un catalyseur d'hydrogénation à une température de 150°C à 400°C et une pression de 0,68 à 27,6 MPa, pour produire le composé de Formule II ; ou
(3) mettre en contact au moins une partie de la fraction solide avec de l'hydrogène et NH₂CH₂CH₂OH ou de l'éthylèneglycol et de l'hydrogène, et éventuellement une source d'ammoniac, en présence d'un catalyseur d'hydrogénation à une température de 150°C à 400°C et une pression de 0,68 à 27,6 MPa, pour produire le composé de Formule III ; et
(l) récupérer les composés de Formule I, de Formule II ou de Formule III

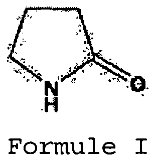

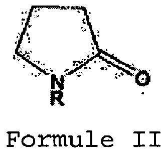

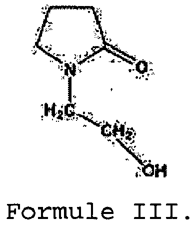

5. Procédés selon l'une quelconque des revendications 1-4, comprenant en outre la mise en contact du composé de Formule I avec de l'acétylène en présence d'un catalyseur basique à une température de 80°C à 250°C et une pression de 0,5 à 25 MPa, pour produire le composé de Formule IV

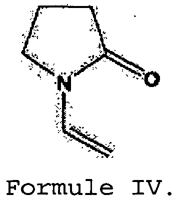

6. Procédés selon l'une quelconque des revendications 1-4, comprenant en outre la déshydratation du composé de Formule III à une température de 100°C à 500°C et une pression de 0,068 à 1,37 MPa, pour produire le composé de Formule IV

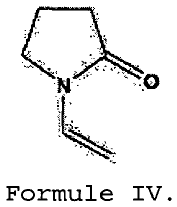

7. Procédés selon l'une quelconque des revendications 1-4, dans lesquels les distillations sont réalisées en présence d'un solvant permettant de séparer l'ammoniac qui est au moins un solvant choisi dans le groupe constitué par le diglyme, le triglyme, le tétraglyme, les sulfoxydes, les amides, les sulfones, le polyéthylène-glycol (PEG), le butoxytriglycol, la N-méthylpyrolidone (NMP), les éthers et la méthyléthylcétone (MEK) ou en présence d'un solvant pouvant être entraîné en azéotrope avec l'eau, qui est au moins un solvant choisi dans le groupe constitué par le toluène, le xylène, le méthylcyclohexane, la méthylisobutylcétone, l'hexane, le cyclohexane et l'heptane.
